(19) Europäisches Patentamt
European Patent Office
Office européen des brevets

(11) Veröffentlichungsnummer: **0 198 191**
**B1**

(12) # EUROPÄISCHE PATENTSCHRIFT

(45) Veröffentlichungstag der Patentschrift: **06.09.89**

(21) Anmeldenummer: **86102767.0**

(22) Anmeldetag: **03.03.86**

(51) Int. Cl.⁴: **C07D 249/08**, C07D 401/06, C07D 403/06, C07D 409/06, C07D 411/06, C07D 413/06, C07D 417/06, C07D 419/06, A01N 43/653

(54) **Piperazinylmethyl-1,2,4-triazolylmethyl-carbinole.**

(30) Priorität: **13.03.85 DE 3508909**

(43) Veröffentlichungstag der Anmeldung: **22.10.86 Patentblatt 86/43**

(45) Bekanntmachung des Hinweises auf die Patenterteilung: **06.09.89 Patentblatt 89/36**

(84) Benannte Vertragsstaaten: **AT BE CH DE FR GB IT LI NL SE**

(56) Entgegenhaltungen:
**EP-A- 0 040 345**
**EP-A- 0 068 144**
**EP-A- 0 110 048**
**EP-A- 0 114 487**
**DE-A- 3 018 866**

(73) Patentinhaber: **BAYER AG, D-5090 Leverkusen 1 Bayerwerk(DE)**

(72) Erfinder: **Holmwood, Graham, Dr., Krutscheider Weg 105, D-5600 Wuppertal 11(DE)**
Erfinder: **Büchel, Karl Heinz, Prof. Dr., Dabringhauser - Strasse 42, D-5093 Burscheid(DE)**
Erfinder: **Brandes, Wilhelm, Dr., Eichendorffstrasse 3, D-5653 Leichlingen(DE)**
Erfinder: **Reinecke, Paul, Dr., Steinstrasse 8, D-5090 Leverkusen 3(DE)**

EP 0 198 191 B1

Anmerkung: Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

**Beschreibung**

Die Erfindung betrifft neue Piperazinylmethyl-1,2,4-triazolmethyl-carbinole, ein Verfahren zu ihrer Herstellung und ihre Verwendung als Pflanzenschutzmittel.

Es ist bereits bekannt, daß bestimmte Triazolylmethyl-carbinole fungizide Eigenschaften besitzen (vgl. DE-A 3 018 866 und EP-A 0 040 345). So lassen sich zum Beispiel 3,3-Dimethyl-2-phenoxymethyl-1-(1,2,4-triazol-1-yl)-butan-2-ol und 2-[(3,4-Dichlorphenoxy)-methyl]-3,3-dimethyl-1-(1,2,4-triazol-1-yl)-butan-2-ol zur Bekämpfung von Pilzen und anderen unerwünschten Mikroorganismen verwenden. Die Wirkung dieser vorbekannten Verbindungen ist jedoch insbesondere bei niedrigen Aufwandmengen und Konzentrationen nicht immer in allen Anwendungsbereichen völlig zufriedenstellend.

Es wurden nun neue Piperazinylmethyl-1,2,4-triazolylmethyl-carbinole der Formel

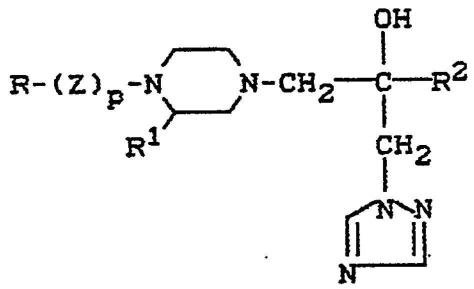
(I)

in welcher

R für geradkettiges oder verzweigtes Alkyl mit 1 bis 12 Kohlenstoffatomen, geradkettiges oder verzweigtes Alkenyl mit 2 bis 12 Kohlenstoffatomen, geradkettiges oder verzweigtes Alkinyl mit 2 bis 12 Kohlenstoffatomen, Alkoxyalkyl mit 1 bis 8 Kohlenstoffatomen im Alkylteil und 1 bis 8 Kohlenstoffatomen im Alkoxyteil, Alkylthioalkyl mit 1 bis 8 Kohlenstoffatomen im Alkylteil und 1 bis 8 Kohlenstoffatomen im Alkylthioteil, Halogenalkyl mit 1 bis 8 Kohlenstoffatomen und 1 bis 17 gleichen oder verschiedenen Halogenatomen, Halogenalkoxyalkyl mit 1 bis 8 Kohlenstoffatomen im Alkylteil und 1 bis 8 Kohlenstoffatomen im Halogenalkoxyteil und 1 bis 17 gleichen oder verschiedenen Halogenatomen, Halogenalkylthioalkyl mit 1 bis 8 Kohlenstoffatomen im Alkylteil und 1 bis 8 Kohlenstoffatomen im Halogenalkylthioteil und 1 bis 17 gleichen oder verschiedenen Halogenatomen, für gegebenenfalls ein- oder mehrfach, gleichartig oder verschieden durch Alkyl mit 1 bis 4 Kohlenstoffatomen substituiertes Cycloalkyl mit 3 bis 7 Ring-Kohlenstoffatomen, für Aryl mit 6 bis 10 Kohlenstoffatomen, Aralkyl mit 6 bis 10 Kohlenstoffatomen im Arylteil und 1 bis 4 Kohlenstoffatomen im Alkylteil, Aryloxyalkyl mit 6 bis 10 Kohlenstoffatomen im Aryloxyteil und 1 bis 4 Kohlenstoffatomen im Alkylteil oder für Arylthioalkyl mit 6 bis 10 Kohlenstoffatomen im Arylthioteil und 1 bis 4 Kohlenstoffatomen im Alkylteil steht, wobei jeder der zuvor genannten Aryl-Reste einfach oder mehrfach, gleichartig oder verschieden substituiert sein kann durch Halogen, geradkettiges oder verzweigtes Alkyl mit 1 bis 4 Kohlenstoffatomen, Alkoxy mit 1 bis 4 Kohlenstoffatomen, Alkylthio mit 1 bis 4 Kohlenstoffatomen, Halogenalkyl mit 1 bis 4 Kohlenstoffatomen und 1 bis 9 gleichen oder verschiedenen Halogenatomen, Halogenalkoxy mit 1 bis 4 Kohlenstoffatomen und 1 bis 9 gleichen oder verschiedenen Halogenatomen, Halogenalkylthio mit 1 bis 4 Kohlenstoffatomen und 1 bis 9 gleichen oder verschiedenen Halogenatomen, Cycloalkyl mit 3 bis 7 Ring-Kohlenstoffatomen, gegebenenfalls durch Halogen und/oder Alkyl mit 1 bis 4 Kohlenstoffatomen substituiertes Phenyl, gegebenenfalls durch Halogen und/oder Alkyl mit 1 bis 4 Kohlenstoffatomen substituiertes Phenylalkyl mit 1 bis 4 Kohlenstoffatomen im Alkylteil, gegebenenfalls durch Halogen und/oder Alkyl mit 1 bis 4 Kohlenstoffatomen substituiertes Phenylalkoxy mit 1 bis 4 Kohlenstoffatomen im Alkoxyteil sowie gegebenenfalls durch Halogen und/oder Alkyl mit 1 bis 4 Kohlenstoffatomen substituiertes Phenoxyalkyl mit 1 bis 4 Kohlenstoffatomen im Alkylteil,

$R^1$ für Wasserstoff oder für geradkettiges oder verzweigtes Alkyl mit 1 bis 4 Kohlenstoffatomen steht,

$R^2$ für geradkettiges oder verzweigtes Alkyl mit 1 bis 8 Kohlenstoffatomen, Alkenyl mit 2 bis 8 Kohlenstoffatomen, für gegebenenfalls einfach oder mehrfach durch Alkyl mit 1 bis 2 Kohlenstoffatomen substituiertes Cycloalkyl mit 3 bis 7 Ring-Kohlenstoffatomen sowie für Phenyl oder Naphtyl steht, wobei jeder der beiden zuvor genannten Reste einfach oder mehrfach, gleichartig oder verschieden substituiert sein kann durch Halogen, geradkettiges oder verzweigtes Alkyl mit 1 bis 4 Kohlenstoffatomen sowie Halogenalkyl mit 1 oder 2 Kohlenstoffatomen und 1 bis 5 gleichen oder verschiedenen Halogenatomen, wie Fluor- und Chloratomen, und

$R^2$ weiterhin für einen 5- oder 6-gliedrigen, gegebenenfalls benzannellierten Heterocyclus mit 1 bis 3 gleichen oder verschiedenen Heteroatomen, wie Sauerstoff, Stickstoff und Schwefel, steht, wobei der Heterocyclus einfach oder mehrfach, gleichartig oder verschieden substituiert sein kann durch Halogen, Alkyl mit 1 bis 4 Kohlenstoffatomen oder Halogenalkyl mit 1 oder 2 Kohlenstoffatomen und 1 bis 5 gleichen oder verschiedenen Halogenatomen, wie Fluor- oder Chloratomen, und

$R^2$ außerdem für die Reste der Formeln

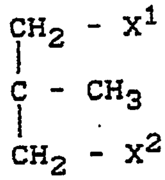 oder 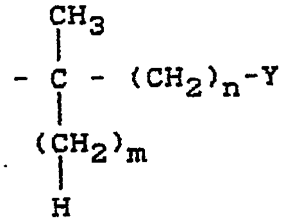 steht,

wobei

$X^1$ für Wasserstoff oder Halogen steht,

$X^2$ für Halogen steht,

Y für geradkettiges oder verzweigtes Alkyl mit 1 bis 6 Kohlenstoffatomen, Alkoxy mit 1 bis 6 Kohlenstoffatomen, Alkylthio mit 1 bis 6 Kohlenstoffatomen, Halogenalkoxy mit 1 bis 6 Kohlenstoffatomen und 1 bis 9 gleichen oder verschiedenen Halogenatomen, Halogenalkylthio mit 1 bis 6 Kohlenstoffatomen und 1 bis 9 gleichen oder verschiedenen Halogenatomen, Alkenyl mit 2 bis 6 Kohlenstoffatomen, geradkettiges oder verzweigtes Alkoxycarbonyl mit 1 bis 4 Kohlenstoffatomen im Alkoxyteil, Cyano sowie für Phenyl, Phenyloxy, Pyridyloxy, Phenylthio, Phenylalkoxy mit 1 bis 4 Kohlenstoffatomen in der Aloxygruppe und Phenylalkylthio mit 1 bis 4 Kohlenstoffatomen in der Alkylthiogruppe steht, wobei jeder der zuvor genannten Phenyl- bzw. Pyridyl-Reste einfach oder mehrfach, gleichartig oder verschieden substituiert sein kann durch Halogen, Alkyl mit 1 bis 4 Kohlenstoffatomen, Alkoxy mit 1 oder 2 Kohlenstoffatomen, Alkylthio mit 1 oder 2 Kohlenstoffatomen, Halogenalkyl mit 1 oder 2 Kohlenstoffatomen und 1 bis 5 gleichen oder verschiedenen Halogenatomen, wie Fluor- und Chloratomen, Halogenalkoxy mit 1 oder 2 Kohlenstoffatomen und 1 bis 5 gleichen oder verschiedenen Halogenatomen, wie Fluor- und Chloratomen, Halogenalkylthio mit 1 oder 2 Kohlenstoffatomen und 1 bis 5 gleichen oder verschiedenen Halogenatomen, wie Fluor- und Chloratomen, Cycloalkyl mit 3 bis 7 Kohlenstoffatomen, Dialkylamino mit 1 bis 4 Kohlenstoffatomen in jedem Alkylteil, Cyano, Nitro sowie geradkettiges oder verzweigtes Alkoxycarbonyl mit 1 bis 4 Kohlenstoffatomen in Alkoxyteil, und

m für eine Zahl 0 oder 1 steht,

n für eine Zahl 0, 1 oder 2 steht,

Z für die 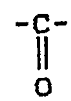 oder die 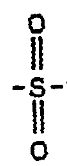-Gruppe steht und

p für eine Zahl 0 oder 1 steht,

sowie die Säureadditionssalze der Halogenwasserstoffsäuren, Phosphorsäure, Salpetersäure, mono-, bi- und trifunktionelle Carbonsäuren und Hydroxycarbonsäuren sowie Sulfonsäuren, und Metallsalzkomplexe mit Salzen von Metallen der II. bis IV. Haupt- und der I. und II. sowie IV. bis VIII. Nebengruppe des Periodensystems der Elemente gefunden.

Weiterhin wurde gefunden, daß man die neuen Piperazinylmethyl-1,2,4-triazolylmethyl-carbinole der Formel (I) sowie deren oben genannte Säureadditionssalze und Metallsalzkomplexe erhält, wenn man Triazolylmethyloxirane der Formel

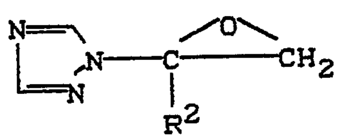 (II)

in welcher

$R^2$ die oben angegebene Bedeutung hat, mit substituierten Piperazinen der Formel

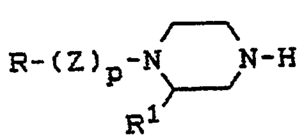 (III)

in welcher

R, $R^1$, Z und p die oben angegebene Bedeutung haben, gegebenenfalls in Gegenwart eines Verdünnungsmittels und gegebenenfalls in Gegenwart eines Katalysators umsetzt und gegebenenfalls anschließend eine Halogenwasserstoffsäure, Phosphorsäure, Salpetersäure, mono-, oder trifunktionelle Carbonsäure, Hydroxycarbonsäure oder Sulfonsäure oder ein Salz von Metallen der II. bis IV. Haupt- oder der I. oder II. oder IV. bis VIII. Nebengruppe des Periodensystems der Elemente addiert.

Schließlich wurde gefunden, daß die neuen Piperazinylmethyl-1,2,4-triazolylmethyl-carbinole der Formel (I) sowie deren oben genannte Säureadditions-Salze und Metallsalz-Komplexe sehr gute fungizide Eigenschaften besitzen.

Überraschenderweise zeigen die erfindungsgemäßen Piperazinylmethyl-1,2,4-triazolylmethyl-carbinole der Formel (I) eine erheblich besser fungizide Wirksamkeit als 3,3-Dimethyl-2-phenoxymethyl-1-(1,2,4-triazol-1-yl)-butan-2-ol und 2-[(3,4-Dichlorphenoxy)-methyl]-3,3-dimethyl-1-(1,2,4-triazol-1-yl)-butan-2-ol, welches chemisch und wirkungsmäßig naheliegende, aus dem Stand der Technik vorbekannte Stoffe sind.

Die erfindungsgemäßen Piperazinylmethyl-1,2,4-triazolylmethyl-carbinole sind durch die Formel (I) allgemein definiert.

Besonders bevorzugt sind Verbindungen der Formel (I), in welchen

R für geradkettiges oder verzweigtes Alkyl mit 1 bis 8 Kohlenstoffatomen, geradkettiges oder verzweigtes Alkenyl mit 2 bis 8 Kohlenstoffatomen, geradkettiges oder verzweigtes Alkinyl mit 2 bis 8 Kohlenstoffatomen, Alkoxyalkyl mit 1 bis 4 Kohlenstoffatomen im Alkylteil und 1 bis 4 Kohlenstoffatomen im Alkoxyteil, Alkylthioalkyl mit 1 bis 4 Kohlenstoffatomen im Alkylteil und 1 bis 4 Kohlenstoffatomen im Alkylthioteil, Halogenalkyl mit 1 bis 4 Kohlenstoffatomen und 1 bis 9 gleichen oder verschiedenen Halogenatomen, Halogenalkoxyalkyl mit 1 bis 4 Kohlenstoffatomen im Alkylteil und 1 bis 4 Kohlenstoffatomen im Halogenalkoxyteil und 1 bis 9 gleichen oder verschiedenen Halogenatomen, Halogenalkylthioalkyl mit 1 bis 4 Kohlenstoffatomen im Alkylteil und 1 bis 4 Kohlenstoffatomen im Halogenalkylthioteil und 1 bis 9 gleichen oder verschiedenen Halogenatomen, für jeweils gegebenenfalls ein- bis dreifach, gleich oder verschieden durch Methyl, Ethyl, n- oder i-Propyl sowie n-, i-, s- oder t-Butyl substituiertes Cyclopropyl, Cyclopentyl oder Cyclohexyl, sowie für Phenylethyl, Benzyl, Phenoxymethyl, Phenoxyethyl, Phenylthiomethyl, Phenylthioethyl, Phenyl oder Naphtyl steht, wobei jeder der zuvor genannten aromatischen Reste ein- bis dreifach, gleichartig oder verschieden substituiert sein kann durch Fluor, Chlor, Brom, Iod, Methyl, Ethyl, n- oder i-Propyl, n-, i-, s- oder t-Butyl Methoxy, Ethoxy, n- oder i-Propoxy, Methylthio, Ethylthio, Trifluormethyl, Trifluormethoxy, Trifluormethylthio, Cyclopropyl, Cyclopentyl, Cyclohexyl sowie Phenyl, Benzyl, Benzyloxy und Phenoxymethyl, wobei jeder der 4 letztgenannten Reste im Phenylteil ein- bis dreifach, gleichartig oder verschieden substituiert sein kann durch Fluor, Chlor, Brom, Methyl, Ethyl und/oder Isopropyl,

$R^1$ für Wasserstoff, Methyl, Etyl, n- oder i-Propyl steht,

$R^2$ für Methyl, Ethyl, n- oder i-Propyl, n-, i-, s-, oder t-Butyl, i-Pentyl, für jeweils gegebenenfalls ein- bis dreifach gleich oder verschieden durch Methyl und/oder Ethyl substituiertes Cyclopropyl, Cyclopentyl oder Cyclohexyl, sowie für Phenyl oder Naphtyl steht, wobei jeder der beiden zuvor genannten Reste ein- bis dreifach, gleichartig oder verschieden substituiert sein kann durch Fluor, Chlor, Brom, Iod, Methyl, Ethyl, n-Propyl, Isopropyl, Trifluormethyl und Trichlormethyl, Trifluormethoxy, und

$R^2$ ferner für jeweils gegebenenfalls ein- bis dreifach, gleichartig oder verschieden substituiertes Pyridyl, Pyrimidyl oder Thienyl steht, wobei Fluor, Chlor, Brom, Iod, Methyl, Ethyl, n-Propyl, iso-Propyl, Trifluormethyl, Trifluormethoxy und Trichlormethyl als Substituenten genannt seien,

$R^2$ außerdem für die Reste der Formeln

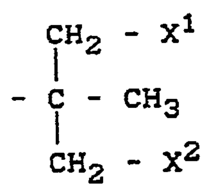

oder

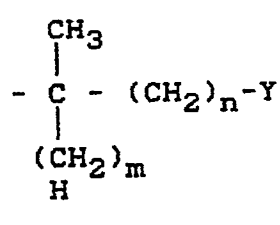

steht,

wobei

$X^1$ für Wasserstoff oder Halogen steht,

$X^2$ für Halogen steht,

Y für Methyl, Ethyl, n- oder i-Propyl, Methoxy, Ethoxy, Methylthio, Ethylthio, Trifluormethoxy, Trifluormethylthio, Vinyl, Allyl, Methoxycarbonyl, Ethoxycarbonyl, Cyano sowie für Phenyl, Phenoxy, Phenylthio, Benzyloxy und Benzylthio steht, wobei jeder der fünf letztgenannten Reste im Phenylteil ein- bis dreifach, gleichartig oder verschieden substituiert sein kann durch Fluor, Chlor, Brom, Methyl, Ethyl, Methoxy, Methylthio, Trifluormethyl, Trifluormethoxy, Trifluormethylthio, Dimethylamino, Diethylamino, Methoxycarbonyl, Ethoxycarbonyl, Cyano oder Nitro, und

Y außerdem für gegebenenfalls durch Chlor substituiertes Pyridoxy steht,

m für eine Zahl 0 oder 1 steht,

n für eine Zahl 0, 1 oder 2 steht,

Z

für die $-\underset{\underset{O}{\|}}{C}-$ oder die $-\overset{\overset{O}{\|}}{\underset{\underset{O}{\|}}{S}}-$Gruppe steht und

p für eine Zahl 0 oder 1 steht.

Besonders bevorzugte erfindungsgemäße Verbindungen sind auch Additionsprodukte aus Piperazinylmethyl-1,2,4-triazolylmethyl-carbinolen der Formel (I), in denen die Substituenten R, $R^1$, $R^2$ und Z sowie der Index p die als besonders bevorzugt genannten Bedeutungen haben, und Chlorwasserstoffsäure, Bromwasserstoffsäure, Phosphorsäure, Salpetersäure, Essigsäure, Maleinsäure, Bernsteinsäure, Fumarsäure, Weinsäure, Zitronensäure, Salizylsäure, Sorbinsäure, Milchsäure, p-Toluolsulfonsäure und 1,5-Naphthalindisulfonsäure.

Außerdem besonders bevorzugte erfindungsgemäße Verbindungen sind Additionsprodukte aus Piperazinylmethyl-1,2,4-triazolylmethyl-carbinolen der Formel (I), in denen die Substituenten R, $R^1$, $R^2$ und Z sowie der Index p die als besonders bevorzugt genannten Bedeutungen haben, und Salzen des Kupfers, Zinks, Mangans, Magnesiums, Zinns, Eisens und des Nickels.

Als Anionen dieser Salze kommen solche in Betracht, die sich von solchen Säuren ableiten, die zu physiologisch verträglichen Additionsprodukten führen. Besonders bevorzugte derartige Säuren sind in diesem Zusammenhang die Halogenwasserstoffsäuren, wie z.B. die Chlorwasserstoffsäure und die Bromwasserstoffsäure, ferner Phosphorsäure, Salpetersäure und Schwefelsäure.

Im einzelnen seien außer den bei den Herstellungsbeispielen genannten Verbindungen die folgenden Piperazinylmethyl-1,2,4-triazolylmethyl-carbinole der Formel (I) genannt:

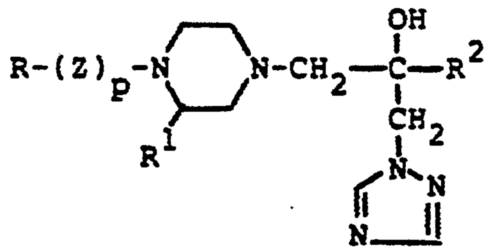

(I)

| R | $R^1$ | $R^2$ | Z | p |
|---|---|---|---|---|
| 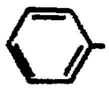 | $CH_3$ | $(CH_3)_3C-$ | - | 0 |
| 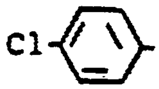 | $CH_3$ | $(CH_3)_3C-$ | - | 0 |
| 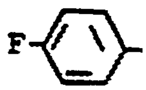 | $CH_3$ | $(CH_3)_3C-$ | - | 0 |
| 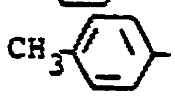 | $CH_3$ | $(CH_3)_3C-$ | - | 0 |
| 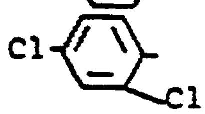 | $CH_3$ | $(CH_3)_3C-$ | - | 0 |
| 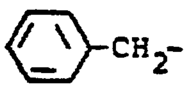 | $CH_3$ | $(CH_3)_3C-$ | - | 0 |
| 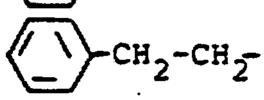 | $CH_3$ | $(CH_3)_3C-$ | - | 0 |
| 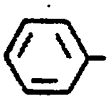 | $CH_3$ | $(CH_3)_3C-$ | 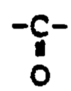 | 1 |
| 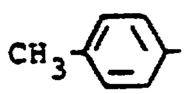 | H | $(CH_3)_3C-$ | $-SO_2-$ | 1 |
| 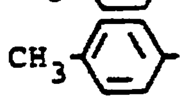 | $CH_3$ | $(CH_3)_3C-$ | $-SO_2-$ | 1 |
| 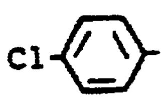 | $CH_3$ | $(CH_3)_3C-$ | $-SO_2-$ | 1 |
| $(CH_3)_2CH-$ | H | 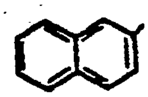 | 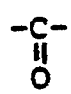 | 1 |

| R | $R^1$ | $R^2$ | Z | p |
|---|---|---|---|---|
| 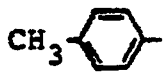 | H | 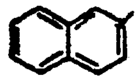 | 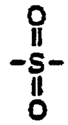 | 1 |
| 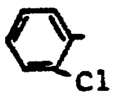 | $CH_3$ | 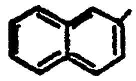 | - | 0 |
| $CH_2=CHCH_2-$ | H | 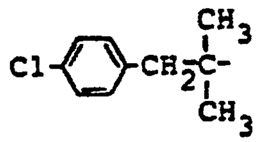 | 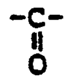 | 1 |
| 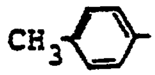 | H | 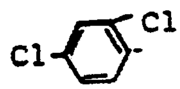 | 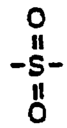 | 1 |
| 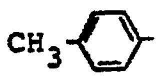 | H | 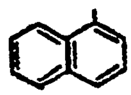 | 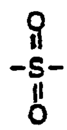 | 1 |
| 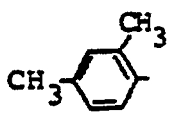 | H | 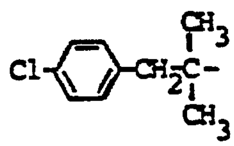 | 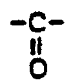 | 1 |
| 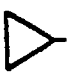 | H | 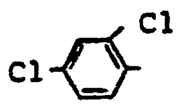 | 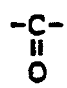 | 1 |
| $(CH_3)_3C-C\equiv C-$ | H | 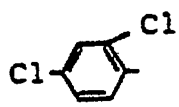 | 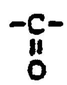 | 1 |
| $(CH_3)_2CH-$ | H | 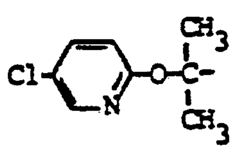 | 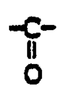 | 1 |
| $(CH_3)_2CH-$ | H | 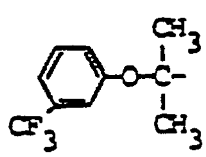 | 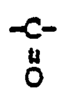 | 1 |
| $(CH_3)_2CH-$ | H | 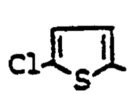 | 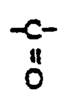 | 1 |

| R | $R^1$ | $R^2$ | Z | p |
|---|---|---|---|---|
| 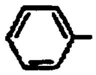 | H | 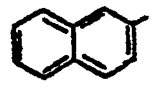 | 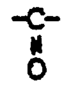 | 1 |
| $CH_3OCH_2-$ | H | 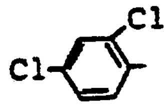 | 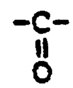 | 1 |
| $CF_3OCH_2-$ | H | 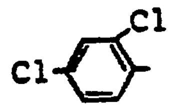 | 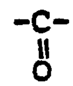 | 1 |
| $(CH_3)_2CH-$ | H | 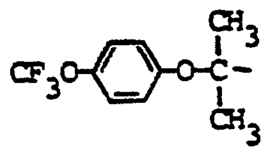 | 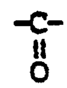 | 1 |
| 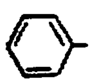 | H | 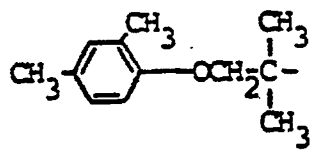 | 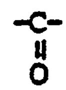 | 1 |
| 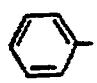 | H | 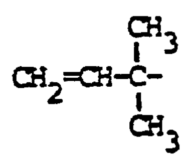 | 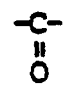 | 1 |

Verwendet man beispielsweise 2-t-Butyl-2-(1,2,4-triazol-1-yl)methyl-oxiran und 1-Phenyl-piperazin als Ausgangsstoffe, so läßt sich der Reaktionsablauf des erfindungsgemäßen Verfahrens durch das folgende Formelschema darstellen:

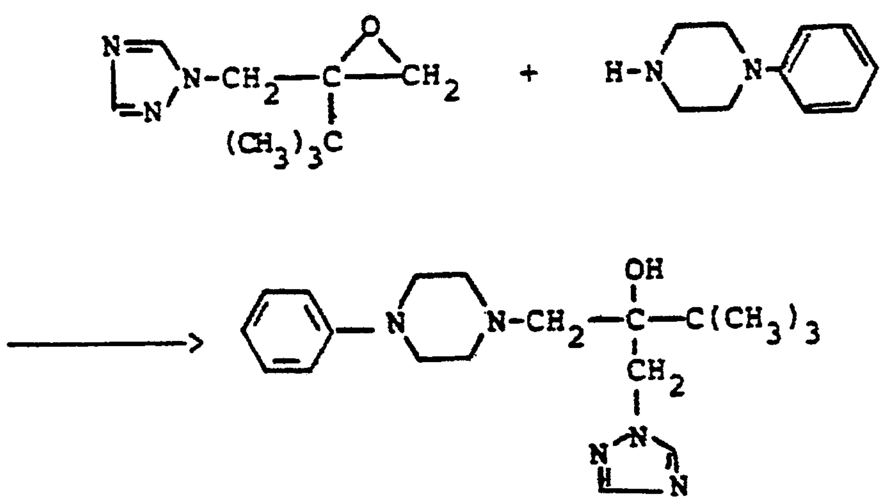

Die zur Durchführung des erfindungsgemäßen Verfahrens als Ausgangsstoffe benötigten Triazolylmethyloxirane sind durch die Formel (II) allgemein definiert. Bevorzugt sind Verbindungen der Formel

(II), bei welchen $R^2$ für diejenigen Reste steht, die schon im Zusammenhang mit der Beschreibung der erfindungsgemäßen Stoffe der Formel (I) als bevorzugt für diesen Substituenten genannt wurden.

Die Triazolylmethyloxirane der Formel (II) sind bekannt (vgl. DE-A 3 111 238) oder lassen sich nach prinzipiell bekannten Verfahren in analoger Weise herstellen.

Die zur Durchführung des erfindungsgemäßen Verfahrens weiterhin als Ausgangsstoffe benötigten substituierten Piperazine sind durch die Formel (III) allgemein definiert. In dieser Formel (III) stehen R, $R^1$, Z und p vorzugsweise für diejenigen Substituenten und Indices, die bereits bei der Beschreibung der erfindungsgemäßen Stoffe der Formel (I) als bevorzugt für diese Reste und Indices genannt wurden.

Die Piperazine der Formel (III) sind allgemein bekannte Verbindungen der organischen Chemie oder lassen sich nach allgemein bekannten Methoden in üblicher Art und Weise herstellen (vgl. US-A 4 258 188).

Als Verdünnungsmittel zur Durchführung des erfindungsgemäßen Verfahrens kommen inerte organische Lösungsmittel in Frage.

Hierzu gehören insbesondere aliphatische oder aromatische, gegebenenfalls halogenierte Kohlenwasserstoffe, wie beispielsweise Benzin, Benzol, Toluol, Xylol, Chlorbenzol, Petrolether, Hexan, Cyclohexan, Dichlormethan, Chloroform, Tetrachlorkohlenstoff, Ether, wie Diethylether, Dioxan, Tetrahydrofuran oder Ethylenglykoldimethyl- oder -diethylether, Nitrile, wie Acetonitril oder Propionitril, Amide, wie Dimethylformamid, Dimethylacetamid und N-Methylformamid, N-Methylpyrrolidon oder Hexamethylphosphorsäuretriamid, Ester, wie Essigsäureethylester, Sulfoxide, wie Dimethylsulfoxid, oder Alkohole, wie Methanol, Ethanol, Propanol oder Butanol.

Das erfindungsgemäße Verfahren kann gegebenenfalls in Gegenwart eines Katalysators durchgeführt werden. Als solche kommen insbesondere anorganische oder organische Basen in Frage.

Hierzu gehören vorzugsweise Alkalicarbonate, wie z.B. Natrium- und Kaliumcarbonat, Alkalihydroxide, wie z.B. Natriumhydroxid; Alkalialkoholate, wie z.B. Natrium- und Kalium-methylat und -ethylat; Alkalihydride, wie z.B. Natriumhydrid; sowie niedere tertiäre Alkylamine, Cycloalkylamine und Aralkylamine, wie insbesondere Triethylamin.

Die Reaktionstemperaturen können bei der Durchführung des erfindungsgemäßen Verfahrens in einem größeren Bereich variiert werden. Im allgemeinen arbeitet man bei Temperaturen zwischen 20°C und 180°C, vorzugsweise zwischen 60°C und 150°C.

Das erfindungsgemäße Verfahren kann unter Normaldruck oder unter erhöhtem Druck durchgeführt werden. Bei der Durchführung unter erhöhtem Druck arbeitet man im allgemeinen zwischen 1 und 50 bar, vorzugsweise zwischen 1 und 25 bar.

Zur Durchführung des erfindungsgemäßen Verfahrens setzt man pro Mol Triazolylmethyloxiran der Formel (II) im allgemeinen 1,0 bis 5,0 Mol, vorzugsweise 1,0 bis 2,0 Mol an substituiertem Piperazin der Formel (III) und gegebenenfalls 0,1 bis 2,0 Mol an Katalysator ein. Die Reaktionsdurchführung, Aufarbeitung und Isolierung der Reaktionsprodukte der Formel (I) erfolgt nach allgemein üblichen Verfahren.

Zur Herstellung von Säureadditions-Salzen der Verbindungen der Formel (I) kommen alle diejenigen Säuren in Frage, die zu physiologisch verträglichen Salzen führen. Vorzugsweise verwendbar sind diejenigen Säuren, die bereits im Zusammenhang mit der Beschreibung der erfindungsgemäßen Stoffe als bevorzugt zu addierende Säuren genannt wurden.

Die Säureadditions-Salze der Verbindungen der Formel (I) lassen sich in einfacher Weise nach üblichen Salzbildungsmethoden herstellen. Im allgemeinen vefährt man so, daß man eine Verbindung der Formel (I) in einem geeigneten inerten Verdünnungsmittel löst und dann eine Säure hinzufügt. Die Isolierung erfolgt in bekannter Weise, zum Beispiel dadurch, daß man das Salz abfiltriert und gegebenenfalls durch Waschen mit einem inerten organischen Lösungsmittel reinigt.

Zur Herstellung von Metallsalz-Komplexen der Verbindungen der Formel (I) kommen vorzugsweise Salze von denjenigen Metallen in Betracht, die bereits im Zusammenhang mit der Beschreibung der erfindungsgemäßen Stoffe als bevorzugt zu addierende Metalle genannt wurden. Als Anionen dieser Metallsalze kommen vorzugsweise Halogenwasserstoffsäuren, wie zum Beispiel Chlorwasserstoffsäure und Bromwasserstoffsäure, ferner Phosphorsäure, Salpetersäure und Schwefelsäure in Frage.

Die Metallsalz-Komplexe von Verbindungen der Formel (I) lassen sich in einfacher Weise nach üblichen Methoden herstellen. Im allgemeinen geht man so vor, daß man ein Metallsalz in Alkohol, wie zum Beispiel Ethanol, löst und dann eine Verbindung der Formel (I) hinzufügt. Die Isolierung erfolgt ebenfalls in bekannter Weise, zum Beispiel dadurch, daß man den Metallsalz-Komplex abfiltriert und gegebenenfalls durch Umkristallisation reinigt.

Die erfindungsgemäßen Wirkstoffe weisen eine starke mikrobizide Wirkung auf und können zur Bekämpfung von unerwünschten Mikroorganismen praktisch eingesetzt werden. Die Wirkstoffe sind für den Gebrauch als Pflanzenschutzmittel geeignet.

Fungizide Mittel im Pflanzenschutz werden eingesetzt zur Bekämpfung von Plasmodiophoromycetes, Oomycetes, Chytridiomycetes, Zygomycetes, Ascomycetes, Basidiomycetes, Deuteromycetes.

Die gute Pflanzenverträglichkeit der Wirkstoffe in den zur Bekämpfung von Pflanzenkrankheiten notwendigen Konzentrationen erlaubt eine Behandlung von oberirdischen Pflanzenteilen, von Pflanz- und Saatgut, und des Bodens.

Dabei lassen sich die erfindungsgemäßen Wirkstoffe mit besoners gutem Erfolg zur Bekämpfung von Getreidekrankheiten, wie beispielsweise gegen die Schaderreger Pyrenophora teres, Cochliobulus sativus, Leptosphaeria nodorum oder Fusarium-Arten, zur Bekämpfung von Gemüsekrankheiten wie beispielsweise gegen den Erreger Uromyces appendiculatus, zur Bekämpfung von Reiskrankheiten, wie beispielsweise gegen den Erreger Pyricularia oryzae sowie zur Bekämpfung von Mehltau- und Rostpilzen einsetzen. Daneben zeigen die erfindungsgemäßen Wirkstoffe auch eine hervorragende antimykotische und antivirale Wirkung und lassen sich beispielsweise zur Bekämpfung von Dermatophyten, Hefen und Schimmelpilzen an Mensch und Tier einsetzen.

Die Wirkstoffe können in die üblichen Formulierungen übergeführt werdem, wie Lösungen, Emulsionen, Suspensionen, Pulver, Schäume, Pasten, Granulate, Aerosole, Wirkstoff-imprägnierte Natur- und synthetische Stoffe, Feinstverkapselungen in polymeren Stoffen und in Hüllmassen für Saatgut, ferner in Formulierungen mit Brennsätzen, wie Räucherpatronen, -dosen, -spiralen u.ä., sowie ULV-Kalt- und Warmnebel-Formulierungen.

Diese Formulierungen werden in bekannter Weise hergestellt, z.B. durch Vermischen der Wirkstoffe mit Streckmitteln, also flüssigen Lösungsmitteln, unter Druck stehenden verflüssigten Gasen und/oder festen Trägerstoffen, gegebenenfalls unter Verwendung von oberflächenaktiven Mitteln, also Emulgiermitteln und/oder Dispergiermitteln und/oder schaumerzeugenden Mitteln. Im Falle der Benutzung von Wasser als Streckmittel können z.B. auch organische Lösungsmittel als Hilfslösungsmittel verwendet werden. Als flüssige Lösungsmittel kommen im wesentlichen in Frage: Aromaten, wie Xylol, Toluol, oder Alkylnaphtaline, chlorierte Aromaten oder chlorierte aliphatische Kohlenwasserstoffe, wie Chlorbenzole, Chlorethylene oder Methylenchlorid, aliphatische Kohlenwasserstoffe, wie Cyclohexan oder Paraffine, z.B. Erdölfraktionen, Alkohole, wie Butanol oder Glycol sowie deren Ether und Ester, Ketone, wie Aceton, Methylethylketon, Methylisobutylketon oder Cyclohexanon, stark polare Lösungsmittel, wie Dimethylformamid und Dimethylsulfoxid, sowie Wasser; mit verflüssigten gasförmigen Streckmitteln oder Trägerstoffen sind solche Flüssigkeiten gemeint, welche bei normaler Temperatur und unter Normaldruck gasförmig sind, z.B. Aerosol-Treibgase, wie Halogenkohlenwasserstoffe sowie Butan, Propan, Stickstoff und Kohlendioxid; als feste Trägerstoffe kommen in Frage: z.B. natürliche Gesteinsmehle, wie Kaoline, Tonerden, Talkum, Kreide, Quarz, Attapulgit, Montmorillonit oder Diatomeenerde und synthetische Gesteinsmehle, wie hochdisperse Kieselsäure, Aluminiumoxid und Silikate; als feste Trägerstoffe für Granulate kommen in Frage: z.B. gebrochene und fraktionierte natürliche Gesteine wie Calcit, Marmor, Bims, Sepiolith, Dolomit sowie synthetische Granulate aus anorganischen und organischen Mehlen sowie Granulate aus organischem Material wie Sägemehl, Kokosnußschalen, Maiskolben und Tabakstengel; als Emulgier- und/oder schaumerzeugende Mittel kommen in Frage: z.B. nichtionogene und anionische Emulgatoren, wie Polyoxyethylen-Fettsäure-Ester, Polyoxyethylen-Fettalkohol-Ether, z.B. Alkyarylpolyglykol-Ether, Alkylsulfonate, Alkylsulfate, Arylsulfonate sowie Eiweißhydrolysate; als Dispergiermittel kommen in Frage: z.B. Lignin-Sulfitablaugen und Methylcellulose.

Es können in den Formulierungen Haftmittel wie Carboxymethylcellulose, natürliche und synthetische pulvrige, körnige oder latexförmige Polymere verwendet werden, wie Gummiarabicum, Polyvinylalkohol, Polyvinylacetat, sowie natürliche Phospholipide, wie Kephaline und Lecithine und synthetische Phospholipide. Weitere Additive können mineralische und vegetabile Öle sein.

Es können Farbstoffe wie anorganische Pigmente, z.B. Eisenoxid, Titanoxid, Ferrocyanblau und organische Farbstoffe, wie Alizarin-, Azo- und Metallphtalocyaninfarbstoffe und Spurennährstoffe wie Salze von Eisen, Mangan, Bor, Kupfer, Kobalt, Molybdän und Zink verwendet werden.

Die Formulierungen enthalten im allgemeinen zwischen 0,1 und 95 Gewichtsprozent Wirkstoff, vorzugsweise zwischen 0,5 und 90%.

Die erfindungsgemäßen Wirkstoffe können in den Formulierungen oder in den verschiedenen Anwendungsformen in Mischung mit anderen bekannten Wirkstoffen vorliegen, wie Fungiziden, Bakeriziden, Insektiziden, Akariziden, Nematoziden, Herbiziden, Schutzstoffen gegen Vogelfraß, Wuchsstoffen, Pflanzennährstoffen und Bodenstrukturverbesserungsmitteln.

Die Wirkstoffe können als solche, in Form ihrer Formulierungen oder den daraus durch weiteres Verdünnen bereiteten Anwendungsformen, wie gebrauchsfertige Lösungen, Emulsionen, Suspensionen, Pulver, Pasten und Granulate angewendet werden. Die Anwendung geschieht in üblicher Weise, z.B. durch Gießen, Tauchen, Spritzen, Sprühen, Vernebeln, Verdampfen, Injizieren, Verschlämmen, Verstreichen, Stäuben, Streuen, Trockenbeizen, Feuchtbeizen, Naßbeizen, Schlämmbeizen oder Inkrustieren.

Bei der Behandlung von Pflanzenteilen können die Wirkstoffkonzentrationen in den Anwendungsformen in einem größeren Bereich variiert werden. Sie liegen im allgemeinen zwischen 1 und 0,0001 Gew.-%, vorzugsweise zwischen 0,5 und 0,001%.

Bei der Saatgutbehandlung werden in allgemeinen Wirkstoffmengen von 0,001 bis 50 g je Kilogramm Saatgut, vorzugweise 0,01 bis 10 g, benötigt.

Bei der Behandlung des Bodens sind Wirkstoffkonzentrationen von 0,00001 bis 0,1 Gew.-%, vorzugsweise von 0,0001 bis 0,02 Gew.-%, am Wirkungsort erforderlich.

Die Herstellung und die Verwendung der erfindungsgemäßen Stoffe geht aus den folgenden Beispielen hervor.

Herstellungsbeispiele

Beispiel 1

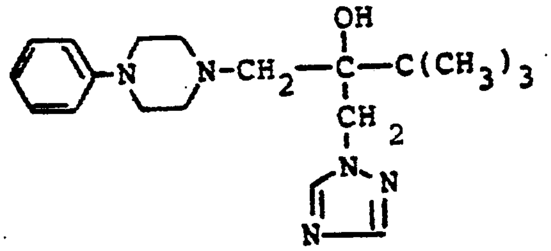

Ein Gemisch aus 16,7 g (0,092 Mol) 2-t-Butyl-1-(1,2,4-triazol-1-yl)-methyl-oxiran und 16,2 (0,1 Mol) 1-Phenylpiperazin in 150 ml Ethanol wird für 15 Stunden unter Rückfluß erhitzt und anschließend unter vermindertem Druck eingeengt. Der Rückstand wird in Essigester aufgenommen, zweimal mit Wasser gewaschen, über Natriumsulfat getrocknet und unter vermindertem Druck vom Lösungsmittel befreit. Nach Umkristallisieren des Rohproduktes aus Petrolether erhält man 19 g (55% der Theorie) an 3,3-Dimethyl-2-[1-(4-phenylpiperazin-1-yl)-methyl]-1-(1,2,4-triazol-1-yl)-butan-2-ol vom Schmelzpunkt 99°C bis 100°C.

In entsprechender Weise und gemäß den allgemeinen Angaben zur Herstellung lassen sich die folgenden Piperazinylmethyl-1,2,4-triazolylmethyl-carbinole der Formel (I) synthetisieren:

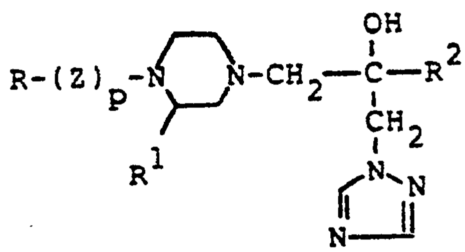

| Bsp. Nr. | R | $R^1$ | $R^2$ | Z | p | physikal. Eigenschaften |
|---|---|---|---|---|---|---|
| 2 | 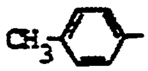 | H | $(CH_3)_3C-$ | - | 0 | Fp 95-96°C |
| 3 | 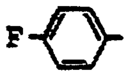 | H | $(CH_3)_3C-$ | - | 0 | Fp 109-110°C |
| 4 | 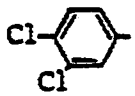 | H | $(CH_3)_3C-$ | - | 0 | Fp 116°C |
| 5 | 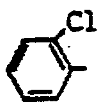 | H | $(CH_3)_3C-$ | - | 0 | Fp 86°C |
| 6 | 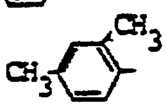 | H | $(CH_3)_3C-$ | - | 0 | Öl |
| 7 | 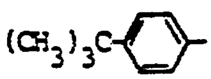 | H | $(CH_3)_3C-$ | - | 0 | Fp 102-103°C |
| 8 | 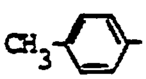 | $CH_3$ | $(CH_3)_3C-$ | - | 0 | Fp 140-141°C |
| 9 | 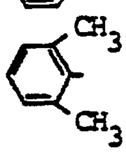 | H | $(CH_3)_3C-$ | - | 0 | Fp 99-100°C |
| 10 | 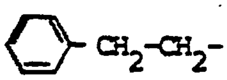 | H | $(CH_3)_3C-$ | - | 0 | Fp 110-111°C |
| 11 | 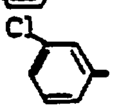 | H | $(CH_3)_3C-$ | - | 0 | Öl |
| 12 | 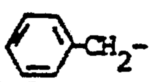 | H | $(CH_3)_3C-$ | - | 0 | Fp 101-102°C |
| 13 | 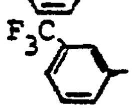 | H | $(CH_3)_3C-$ | - | 0 | Fp 78-79°C |
| 14 | 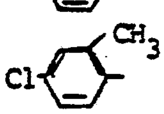 | H | $(CH_3)_3C-$ | - | 0 | Fp 101-102°C |
| 15 | 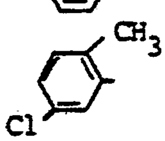 | H | $(CH_3)_3C-$ | - | 0 | Fp 115-116°C |

| Bsp. Nr. | R | $R^1$ | $R^2$ | Z | p | physikal. Eigenschaften |
|---|---|---|---|---|---|---|
| 16 | 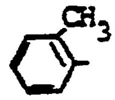 | H | $(CH_3)_3C-$ | - | 0 | Fp 91-92°C |
| 17 | 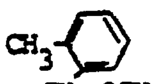 | H | $(CH_3)_3C-$ | - | 0 | Fp 126-127°C |
| 18 | 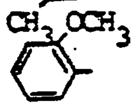 | H | $(CH_3)_3C-$ | - | 0 | Öl |
| 19 | 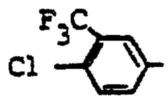 | H | $(CH_3)_3C-$ | - | 0 | Öl |
| 20 | 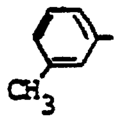 | H | $(CH_3)_3C-$ | - | 0 | Fp 84°C |
| 21 | 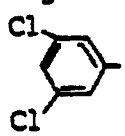 | H | $(CH_3)_3C-$ | - | 0 | Fp 106-107°C |
| 22 | 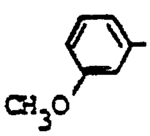 | H | $(CH_3)_3C-$ | - | 0 | Öl |
| 23 | $(CH_3)_2CH-$ | H | $(CH_3)_3C-$ | 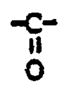 | 1 | Öl |
| 24 | $(CH_3)_2CH-$ | H | 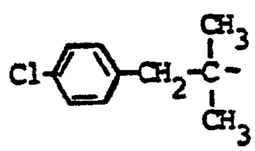 | 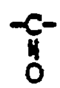 | 1 | Fp 141°C |
| 25 | $C_2H_5$ | H | 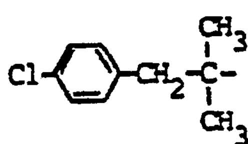 | 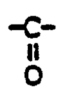 | 1 | Fp 119°C |
| 26 | $CH_3-(CH_2)_3-$ | H | 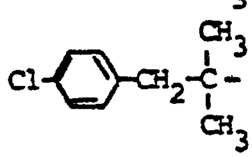 | 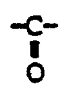 | 1 | 87-89°C |
| 27 | $C_2H_5$ | H | 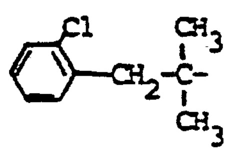 | 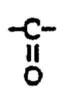 | 1 | Öl |

| Bsp. Nr. | R | $R^1$ | $R^2$ | Z | p | physikal. Eigenschaften |
|---|---|---|---|---|---|---|
| 28 | $(CH_3)_2CH-$ | H | 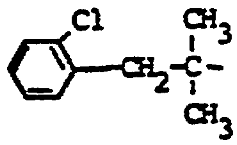 | 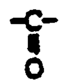 | 1 | Öl |
| 29 | 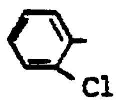 | H | 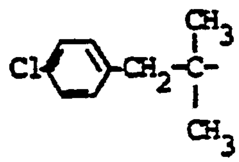 | - | 0 | |
| 30 | 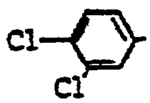 | H | 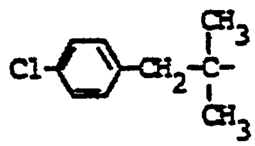 | - | 0 | |
| 31 | $(CH_3)_2CH-CH_2-$ | H | 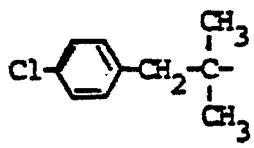 | 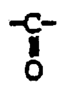 | 1 | Fp 94-96°C |
| 32 | 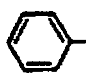 | H | 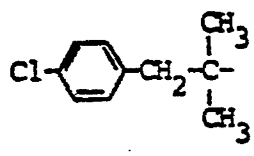 | 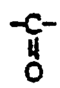 | 1 | Fp 140-141°C |
| 33 | 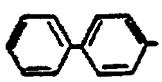 | H | 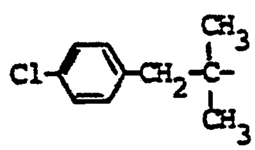 | 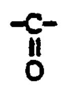 | 1 | |
| 34 | 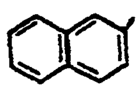 | H | 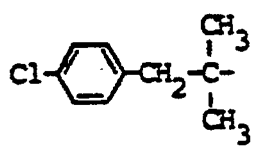 | 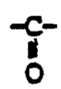 | 1 | |
| 35 | $(CH_3)_2CH-$ | H | 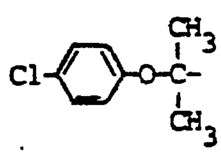 | 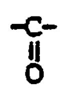 | 1 | |

| Bsp. Nr. | R | $R^1$ | $R^2$ | Z | p | physikal. Eigenschaften |
|---|---|---|---|---|---|---|
| 36 | $(CH_3)_2CH-$ | H | 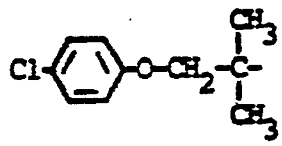 | 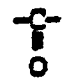 | 1 | |
| 37 | $(CH_3)_2CH-$ | H | 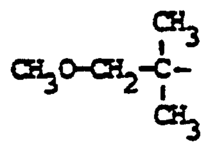 | 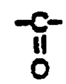 | 1 | |
| 38 | 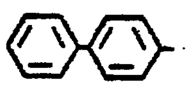 | H | 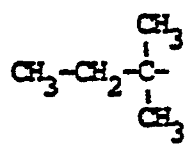 | 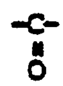 | 1 | |
| 39 | 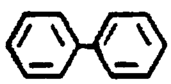 | H | 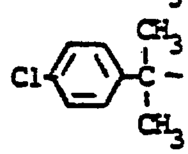 | 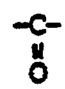 | 1 | |
| 40 | 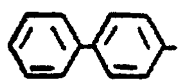 | H | 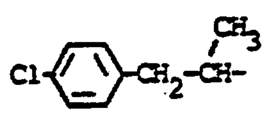 | 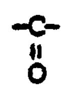 | 1 | |
| 41 | 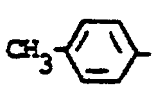 | H | 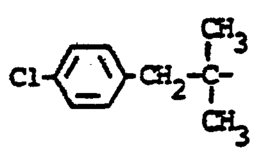 | 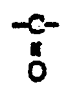 | 1 | |
| 42 | 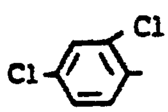 | H | 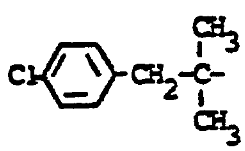 | 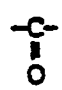 | 1 | |
| 43 | 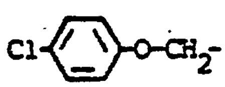 | H | 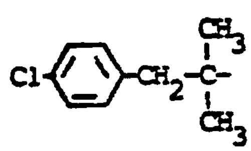 | 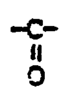 | 1 | |
| 44 | 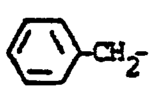 | H | 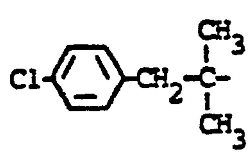 | 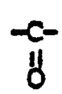 | 1 | |
| 45 | 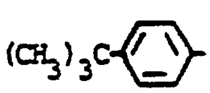 | H | 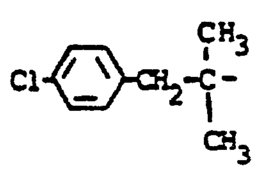 | 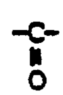 | 1 | |

| Bsp. Nr. | R | $R^1$ | $R^2$ | Z | p | physikal. Eigenschaften |
|---|---|---|---|---|---|---|
| 46 | 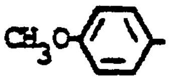 | H | 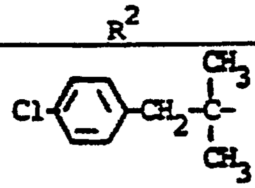 | 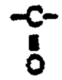 | 1 | |
| 47 | 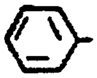 | H | 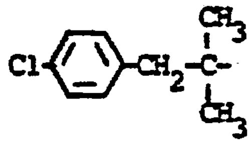 | $-SO_2-$ | 1 | |
| 48 | 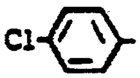 | H | 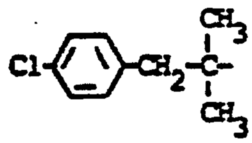 | $-SO_2-$ | 1 | |
| 49 | $(CH_3)_2CH-$ | H | 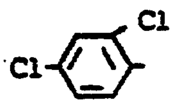 | 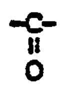 | 1 | |
| 50 | 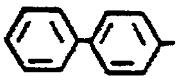 | H | 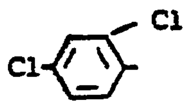 | 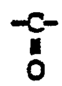 | 1 | |
| 51 | 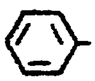 | H | 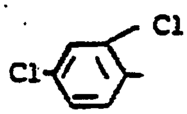 | 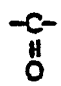 | 1 | |
| 52 | 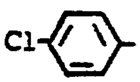 | H | 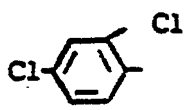 | 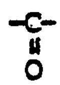 | 1 | |
| 53 | 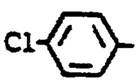 | H | 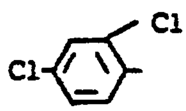 | - | 0 | |
| 54 | 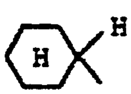 | H | 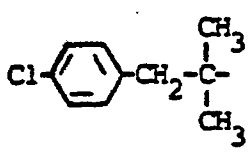 | 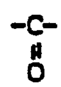 | 1 | |

| Beispiel Nr. | R | $R^1$ | $R^2$ | Z | p | Physikalische Eigenschaften |
|---|---|---|---|---|---|---|
| 55 | $CH_3$ - | H | 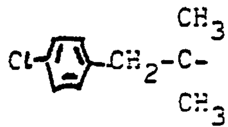 | $-\underset{\overset{\|}{O}}{C}-$ | 1 | Fp 110-113°C |
| 56 | 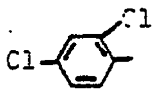 | H | $(CH_3)_3C$- | $-\underset{\overset{\|}{O}}{C}-$ | 1 | Glas |
| 57 | 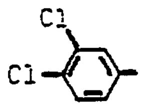 | H | $(CH_3)_3C$- | $-\underset{\overset{\|}{O}}{C}-$ | 1 | Fp 121°C |
| 58 | 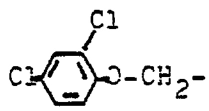 | H | $(CH_3)_3C$ | $-\underset{\overset{\|}{O}}{C}-$ | 1 | Glas |
| 59 | 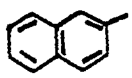 | H | $(CH_3)_3C$ | $-\underset{\overset{\|}{O}}{C}-$ | 1 | Fp 117-9°C |
| 60 | $(CH_3)_3C$-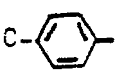 | H | $(CH_3)_3C$ | $-\underset{\overset{\|}{O}}{C}-$ | 1 | Fp 117-8°C |
| 61 | $(CH_3)_2CH$- | H | 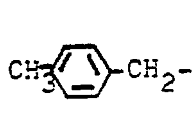 | $-\underset{\overset{\|}{O}}{C}-$ | 1 | Fp 117-8°C |
| 62 | $(CH_3)_2CH-CH_2$ | H | 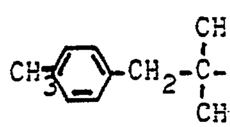 | $-\underset{\overset{\|}{O}}{C}-$ | 1 | Glas |
| 63 | 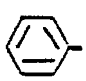 | H | 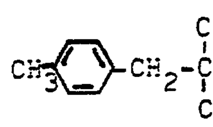 | $-\underset{\overset{\|}{O}}{C}-$ | 1 | Fp 154-5°C |

Anwendungsbeispiele

In den folgenden Anwendungsbeispielen wurden die nachstehend aufgeführten Verbindungen als Vergleichssubstanzen eingesetzt:

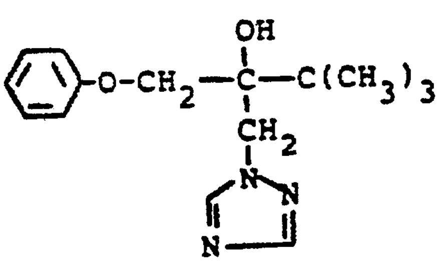

(A)

3,3-Dimethyl-2-phenoxymethyl-1-(1,2,4-triazol-1-yl)-butan-2-ol

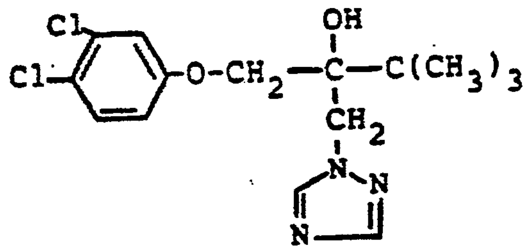

(B)

2-[(3,4-Dichlorphenoxy)-methyl]-3,3-dimethyl-1-(1,2,4-triazol-1-yl)-butan-2-ol (beide bekannt aus DE-A 3 018 866 und aus EP-A 0 040 345).

Beispiel A

Pyrenphora teres-Test (Gerste) / protektiv
Lösungsmittel: 100 Gewichtsteile Dimethylformamid
Emulgator: 0,25 Gewichtsteile Alkylarylpolyglykolether

Zur Herstellung einer zweckmäßigen Wirkstoffzubereitung vermischt man 1 Gewichtsteil Wirkstoff mit den angegebenen Mengen Lösungsmittel und Emulgator und verdünnt das Konzentrat mit Wasser auf die gewünschte Konzentration.

Zur Prüfung auf protektive Wirksamkeit besprüht man junge Pflanzen mit der Wirkstoffzubereitung taufeucht. Nach Antrocknen des Spritzbelages werden die Pflanzen mit einer Konidiensuspension von Pyrenophora teres besprüht. Die Pflanzen verbleiben 48 Stunden bei 20°C und 100% rel. Luftfeuchtigkeit in einer Inkubationskabine.

Die Pflanzen werden in einem Gewächshaus bei einer Temperatur von ca. 20°C und einer relativen Luftfeuchtigkeit von ca. 80% aufgestellt.

7 Tage nach der Inokulation erfolgt die Auswertung.

In diesem Test zeigen die in den Beispielen 5, 6, 7, 8, 9, 11, 13, 14, 15, 19, 20, 21, 24, 25, 26 und 31 offenbarten erfindungsgemäßen Stoffe eine deutlich bessere Wirksamkeit als die Vergleichssubstanz (A).

Beispiel B

Uromyces Test (Buschbohnen) / protektiv
Lösungsmittel: 4,7 Gewichtsteile Aceton
Emulgator: 0,3 Gewichtsteile Alkyl-aryl-polyglykolether

Zur Herstellung einer zweckmäßigen Wirkstoffzubereitung vermischt man 1 Gewichtsteil Wirkstoff mit den angegebenen Mengen Lösungsmittel und Emulgator und verdünnt das Konzentrat mit Wasser auf die gewünschte Konzentration.

Zur Prüfung auf protektive Wirksamkeit bespritzt man junge Pflanzen mit der Wirkstoffzubereitung bis zur Tropfnässe. Nach Antrocknen des Spritzbelages werden die Pflanzen mit einer wäßrigen Uredosporensuspension des Bohnenrosterregers (Uromyces appendiculatus) inokuliert und verbleiben 1 Tag in einer dunklen Feuchtkammer bei 20 bis 22°C und 100% relativer Luftfeuchtigkeit.

Die Pflanzen werden dann unter intensiver Belichtung für 9 Tage bei 20 bis 22°C und einer relativen Luftfeuchtigkeit von 70 bis 80% im Gewächshaus aufgestellt.

10 Tage nach der Inokulation erfolgt die Auswertung.

In diesem Test zeigen die in den Beispielen 4, 11, 13, 14 und 15 offenbarten erfindungsgemäßen Stoffe eine deutlich bessere Wirksamkeit als die Vergleichssubstanz (B).

## Patentansprüche

1. Piperazinylmethyl-1,2,4-triazolyl-methyl-carbinole der Formel (I),

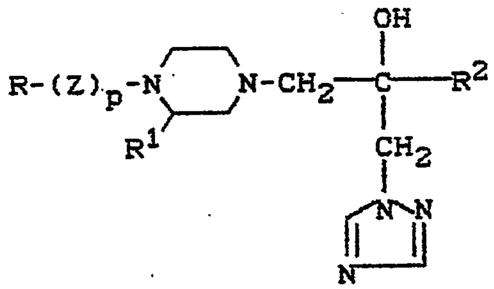

(I)

in welcher
R für geradkettiges oder verzweigtes Alkyl mit 1 bis 12 Kohlenstoffatomen, geradkettiges oder verzweigtes Alkenyl mit 2 bis 12 Kohlenstoffatomen, geradkettiges oder verzweigtes Alkinyl mit 2 bis 12 Kohlenstoffatomen, Alkoxyalkyl mit 1 bis 8 Kohlenstoffatomen im Alkylteil und 1 bis 8 Kohlenstoffatomen im Alkoxyteil, Alkylthioalkyl mit 1 bis 8 Kohlenstoffatomen im Alkylteil und 1 bis 8 Kohlenstoffatomen im Alkylthioteil, Halogenalkyl mit 1 bis 8 Kohlenstoffatomen und 1 bis 17 gleichen oder verschiedenen Halogenatomen, Halogenalkoxyalkyl mit 1 bis 8 Kohlenstoffatomen im Alkylteil und 1 bis 8 Kohlenstoffatomen im Halogenalkoxyteil und 1 bis 17 gleichen oder verschiedenen Halogenatomen, Halogenalkylthioalkyl mit 1 bis 8 Kohlenstoffatomen im Alkylteil und 1 bis 8 Kohlenstoffatomen im Halogenalkylthioteil und 1 bis 17 gleichen oder verschiedenen Halogenatomen, für gegebenenfalls ein- oder mehrfach, gleichartig oder verschieden durch Alkyl mit 1 bis 4 Kohlenstoffatomen substituiertes Cycloalkyl mit 3 bis 7 Ring-Kohlenstoffatomen, für Aryl mit 6 bis 10 Kohlenstoffatomen, Aralkyl mit 6 bis 10 Kohlenstoffatomen im Arylteil und 1 bis 4 Kohlenstoffatomen im Alkylteil, Aryloxyalkyl mit 6 bis 10 Kohlenstoffatomen im Aryloxyteil und 1 bis 4 Kohlenstoffatomen im Alkylteil oder für Arylthioalkyl mit 6 bis 10 Kohlenstoffatomen im Arylthioteil und 1 bis 4 Kohlenstoffatomen im Alkylteil steht, wobei jeder der zuvor genannten Aryl-Reste einfach oder mehrfach, gleichartig oder verschieden substituiert sein kann durch Halogen, geradkettiges oder verzweigtes Alkyl mit 1 bis 4 Kohlenstoffatomen, Alkoxy mit 1 bis 4 Kohlenstoffatomen, Alkylthio mit 1 bis 4 Kohlenstoffatomen, Halogenalkyl mit 1 bis 4 Kohlenstoffatomen und 1 bis 9 gleichen oder verschiedenen Halogenatomen, Halogenalkoxy mit 1 bis 4 Kohlenstoffatomen und 1 bis 9 gleichen oder verschiedenen Halogenatomen, Halogenalkylthio mit 1 bis 4 Kohlenstoffatomen und 1 bis 9 gleichen oder verschiedenen Halogenatomen, Cycloalkyl mit 3 bis 7 Ring-Kohlenstoffatomen, gegebenenfalls durch Halogen und/oder Alkyl mit 1 bis 4 Kohlenstoffatomen substituiertes Phenyl, gegebenenfalls durch Halogen und/oder Alkyl mit 1 bis 4 Kohlenstoffatomen substituiertes Phenylalkyl mit 1 bis 4 Kohlenstoffatomen im Alkylteil, gegebenenfalls durch Halogen und/oder Alkyl mit 1 bis 4 Kohlenstoffatomen substituiertes Phenylalkoxy mit 1 bis 4 Kohlenstoffatomen im Alkoxyteil sowie gegebenenfalls durch Halogen und/oder Alkyl mit 1 bis 4 Kohlenstoffatomen substituiertes Phenoxyalkyl mit 1 bis 4 Kohlenstoffatomen im Alkylteil,
$R^1$ für Wasserstoff oder für geradkettiges oder verzweigtes Alkyl mit 1 bis 4 Kohlenstoffatomen steht.
$R^2$ für geradkettiges oder verzweigtes Alkyl mit 1 bis 8 Kohlenstoffatomen, Alkenyl mit 2 bis 8 Kohlenstoffatomen, für gegebenenfalls einfach oder mehrfach durch Alkyl mit 1 bis 2 Kohlenstoffatomen substituiertes Cycloalkyl mit 3 bis 7 Ring- Kohlenstoffatomen sowie für Phenyl oder Naphthyl steht, wobei jeder der beiden zuvor genannten Reste einfach oder mehrfach, gleichartig oder verschieden substituiert sein kann durch Halogen, geradkettiges oder verzweigtes Alkyl mit 1 bis 4 Kohlenstoffatomen sowie Halogenalkyl mit 1 oder 2 Kohlenstoffatomen und 1 bis 5 gleichen oder verschiedenen Halogenatomen, wie Fluor- und Chloratomen, und
$R^2$ weiterhin für einen 5- oder 6-gliedrigen, gegebenenfalls benzannellierten Heterocyclus mit 1 bis 3 gleichen oder verschiedenen Heteroatomen, wie Sauerstoff, Stickstoff und Schwefel, steht, wobei der Heterocyclus einfach oder mehrfach, gleichartig oder verschieden substituiert sein kann durch Halogen, Alkyl mit 1 bis 4 Kohlenstoffatomen oder Halogenalkyl mit 1 oder 2 Kohlenstoffatomen und 1 bis 5 gleichen oder verschiedenen Halogenatomen, wie Fluor- oder Chloratomen, und
$R^2$ außerdem für die Reste der Formeln

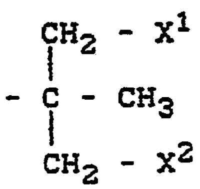

oder

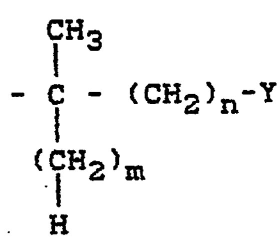

steht,

wobei
$X^1$ für Wasserstoff oder Halogen steht,
$X^2$ für Halogen steht,
Y für geradkettiges oder verzweigtes Alkyl mit 1 bis 6 Kohlenstoffatomen, Alkoxy mit 1 bis 6 Kohlenstoffatomen, Alkylthio mit 1 bis 6 Kohlenstoffatomen, Halogenalkoxy mit 1 bis 6 Kohlenstoffatomen und 1 bis 9 gleichen oder verschiedenen Halogenatomen, Halogenalkylthio mit 1 bis 6 Kohlenstoffatomen und 1 bis 9 gleichen oder verschiedenen Halogenatomen, Alkenyl mit 2 bis 6 Kohlenstoffatomen, geradkettiges oder verzweigtes Alkoxycarbonyl mit 1 bis 4 Kohlenstoffatomen im Alkoxyteil, Cyano sowie für Phenyl, Phenyloxy, Pyridyloxy, Phenylthio, Phenylalkoxy mit 1 bis 4 Kohlenstoffatomen in der Alkoxygruppe und Phenylalkylthio mit 1 bis 4 Kohlenstoffatomen in der Alkylthiogruppe steht, wobei jeder der zuvor genannten Phenyl- bzw. Pyridyl-Reste einfach oder mehrfach, gleichartig oder verschieden substituiert sein kann durch Halogen, Alkyl mit 1 bis 4 Kohlenstoffatomen, Alkoxy mit 1 oder 2 Kohlenstoffatomen, Alkylthio mit 1 oder 2 Kohlenstoffatomen, Halogenalkyl mit 1 oder 2 Kohlenstoffatomen und 1 bis 5 gleichen oder verschiedenen Halogenatomen, wie Fluor- und Chloratomen, Halogenalkoxy mit 1 oder 2 Kohlenstoffatomen und 1 bis 5 gleichen oder verschiedenen Halogenatomen, wie Fluor- und Chloratomen, Halogenalkylthio mit 1 oder 2 Kohlenstoffatomen und 1 bis 5 gleichen oder verschiedenen Halogenatomen, wie Fluor- und Chloratomen, Cycloalkyl mit 3 bis 7 Kohlenstoffatomen, Dialkylamino mit 1 bis 4 Kohlenstoffatomen in jedem Alkylteil, Cyano, Nitro sowie geradkettiges oder verzweigtes Alkoxycarbonyl mit 1 bis 4 Kohlenstoffatomen im Alkoxyteil, und
m für eine Zahl 0 oder 1 steht,
n für eine Zahl 0, 1 oder 2 steht,
Z

```
                                  O
                                  ||
für die -C- oder die -S-Gruppe steht und
         ||          ||
         O           O
```

p für eine Zahl 0 oder 1 steht,
sowie die Säureadditionssalze der Halogenwasserstoffsäuren, Phosphorsäure, Salpetersäure, monobi- und trifunktionelle Carbonsäuren und Hydroxycarbonsäuren sowie Sulfonsäuren, und Metallsalzkomplexe mit Salzen von Metallen der II. bis IV. Haupt- und der I. und II. sowie IV. bis VIII. Nebengruppe des Periodensystems der Elemente.

2. Verfahren zur Herstellung von Piperazinyl-methyl-1, 2, 4-triazolylmethylcarbinolen der Formel

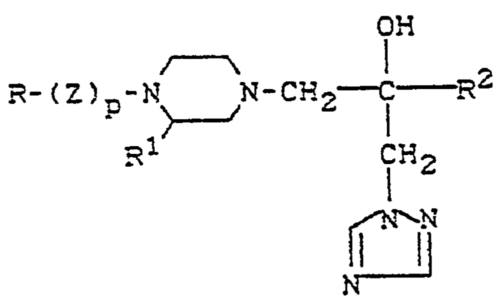

(I)

in welcher
R, $R^1$, $R^2$, Z und p die in Anspruch 1 angegebene Bedeutung haben, sowie von deren Säureadditionssalzen und Metallsalzkomplexen gemäß Anspruch 1, dadurch gekennzeichnet, daß man Triazolylmethyloxirane der Formel

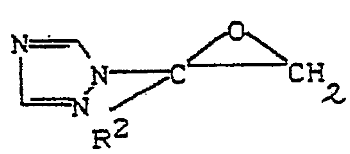

(II)

in welcher
$R^2$ die oben angegebene Bedeutung hat, mit substituierten Piperazinen der Formel

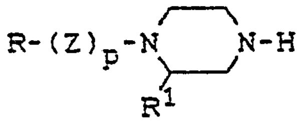

(III)

in welcher
R, R1, z und p die oben angegebene Bedeutung haben, gegebenenfalls in Gegenwart eines Verdünnungsmittels und gegebenenfalls in Gegenwart eines Katalysators umsetzt und gegebenenfalls anschließend eine Halogenwasserstoffsäure, Phosphorsäure, Salpetersäure, mono-, di- oder tri- funktionelle Carbonsäure, Hydroxycarbonsäure oder Sulfonsäure oder ein Salz von Metallen der II. bis IV. Haupt- oder der I. oder II. oder IV. bis VIII. Nebengruppe des Periodensystems der Elemente addiert.

3. Pflanzenschutzmittel, gekennzeichnet durch einen Gehalt an mindestens einem Piperazinylmethyl-1,2,4-triazolylmethyl-carbinol der Formel (I) gemäß Anspruch 1 oder einem Säureadditionssalz bzw. Metallsalzkomplex eines Piperazinylmethyl-1,2,4-triazolylmethyl-carbinols der Formel (I) gemäß Anspruch 1.

4. Verwendung von Piperazinylmethyl-1,2,4-triazolylmethyl-carbinolen der Formel (I) gemäß Anspruch 1 bzw. deren Säureadditionssalzen und Metallsalzkomplexen gemäß Anspruch 1 zum Schutz von Pflanzen.

5. Verfahren zum Schutz von Pflanzen, dadurch gekennzeichnet, daß man Piperazinylmethyl-1,2,4-triazolyl-methyl-carbinole der Formel (I) gemäß Anspruch 1 bzw. deren Säureadditionssalze und Metallsalzkomplexe gemäß Anspruch 1 auf Pflanzen und/oder deren Lebensraum ausbringt.

6. Verfahren zur Herstellung von Pflanzenschutzmitteln, dadurch gekennzeichnet, daß man Piperazinylmethyl-1,2,4-triazolyl-methyl-carbinole der Formel (I) gemäß Anspruch 1 bzw. deren Säureadditionssalze und Metallsalzkomplexe gemäß Anspruch 1 mit Streckmitteln und/oder oberflächenaktiven Stoffen vermischt.

7. Piperazinylmethyl-1,2,4-triazolyl-methyl-carbinol gemäß Anspruch 1 der Formel

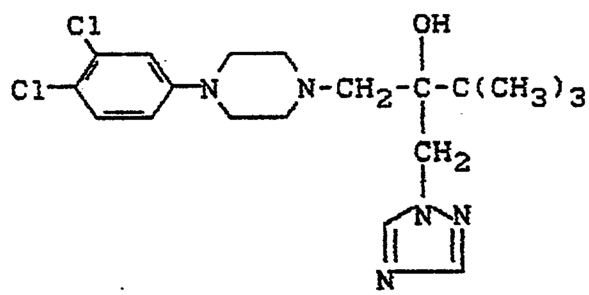

8. Piperazinylmethyl-1,2,4-triazolyl-methyl-carbinol gemäß Anspruch 1 der Formel

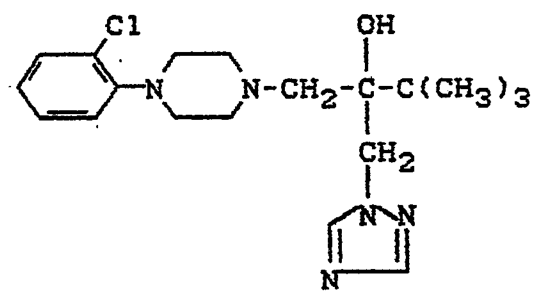

9. Piperazinylmethyl-1,2,4-triazolyl-methyl-carbinol gemäß Anspruch 1 der Formel

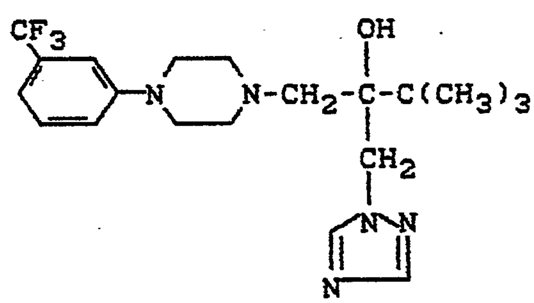

## Claims

1. Piperazinylmethyl-1,2,4-triazolylmethyl-carbinols of the formula (I)

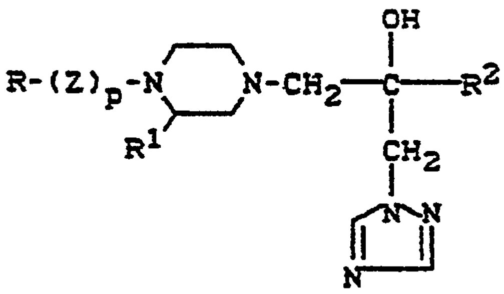

(I)

in which

R represents straight-chain or branched alkyl having 1 to 12 carbon atoms, straight-chain or branched alkenyl having 2 to 12 carbon atoms, straight-chain or branched alkinyl having 2 to 12 carbon atoms, alkoxyalkyl having 1 to 8 carbon atoms in the alkyl part and 1 to 8 carbon atoms in the alkoxy part, alkylthioalkyl having 1 to 8 carbon atoms in the alkyl part and 1 to 8 carbon atoms in the alkylthio part, halogenoalkyl having 1 to 8 carbon atoms and 1 to 17 identical or different halogen atoms, halogenoalkoxyalkyl having 1 to 8 carbon atoms in the alkyl part and 1 to 8 carbon atoms in the halogenoalkoxy part and 1 to 17 identical or different halogen atoms, halogenoalkylthioalkyl having 1 to 8 carbon atoms in the alkyl part and 1 to 8 carbon atoms in the halogenoalkylthio part and 1 to 17 identical or different halogen atoms, cycloalkyl which has 3 to 7 ring carbon atoms and is optionally monosubstituted or polysubstituted by identical or different alkyl radicals of 1 to 4 carbon atoms, or represents aryl having 6 to 10 carbon atoms, aralkyl having 6 to 10 carbon atoms in the aryl part and 1 to 4 carbon atoms in the alkyl part, aryloxyalkyl having 6 to 10 carbon atoms in the aryloxy part and 1 to 4 carbon atoms in the alkyl part, or arylthioalkyl having 6 to 10 carbon atoms in the arylthio part and 1 to 4 carbon atoms in the alkyl part, it being possible for each of the abovementioned aryl radicals to be monosubstituted or polysubstituted by identical or different substituents from amongst halogen, straight-chain or branched alkyl having 1 to 4 carbon atoms, alkoxy having 1 to 4 carbon atoms, alkylthio having 1 to 4 carbon atoms, halogenoalkyl having 1 to 4 carbon atoms and 1 to 9 identical or different halogen atoms, halogenoalkoxy having 1 to 4 carbon atoms and 1 to 9 identical or different halogen atoms, halogenoalkylthio having 1 to 4 carbon atoms and 1 to 9 identical or different halogen atoms, cycloalkyl having 3 to 7 ring carbon atoms, phenyl which is optionally substituted by halogen and/or alkyl having 1 to 4 carbon atoms, phenylalkyl which has 1 to 4 carbon atoms in the alkyl part and is optionally substituted by halogen and/or alkyl having 1 to 4 carbon atoms, phenylalkoxy which has 1 to 4 carbon atoms in the alkoxy part and is optionally substituted by halogen and/or alkyl having 1 to 4 carbon atoms, and phenoxyalkyl which has 1 to 4 carbon atoms in the alkyl part and is optionally substituted by halogen and/or alkyl having 1 to 4 carbon atoms,

$R^1$ represents hydrogen or straight-chain or branched alkyl having 1 to 4 carbon atoms,

$R^2$ represents straight-chain or branched alkyl having 1 to 8 carbon atoms, straight-chain or branched alkenyl having 2 to 8 carbon atoms, or represents cycloalkyl which has 3 to 7 ring carbon atoms and is optionally monosubstituted or polysubstituted by alkyl having 1 or 2 carbon atoms, or represents phenyl or naphthyl, it being possible for each of the two radicals mentioned above to be monosubstituted or polysubstituted by identical or different substituents from amongst halogen, straight-chain or branched alkyl having 1 to 4 carbon atoms and halogenoalkyl having 1 or 2 carbon atoms and 1 to 5 identical or different halogen atoms, such as fluorine atoms and chlorine atoms, and

$R^2$ furthermore represents a 5-membered or 6-membered, optionally benzofused heterocyclic structure having 1 to 3 identical or different hetero atoms, such as oxygen, nitrogen and sulphur, it being possible for the heterocyclic structure to be monosubstituted or polysubstituted by identical or different substituents from amongst halogen, alkyl having 1 to 4 carbon atoms and halogenoalkyl having 1 or 2 carbon atoms and 1 to 5 identical or different halogen atoms, such as fluorine atoms or chlorine atoms, and

$R^2$ furthermore represents the radicals of the formulae

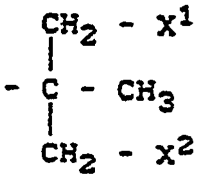

or

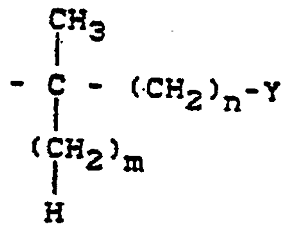

wherein

$X^1$ represents hydrogen or halogens,

$X^2$ represents halogen,

Y represents straight-chain or branched alkyl having 1 to 6 carbon atoms, alkoxy having 1 to 6 carbon atoms, alkylthio having 1 to 6 carbon atoms, halogenoalkoxy having 1 to 6 carbon atoms and 1 to 9 identical or different halogen atoms, halogenoalkylthio having 1 to 6 carbon atoms and 1 to 9 identical or different halogen atoms, alkenyl having 2 to 6 carbon atoms, straight-chain or branched alkoxycarbonyl having 1 to 4 carbon atoms in the alkoxy part, or cyano, or represents phenyl, phenoxy, pyridyloxy, phenylthio, phenylalkoxy having 1 to 4 carbon atoms in the alkoxy group and phenylalkylthio having 1 to 4 carbon atoms in the alkylthio group, it being possible for each of the abovementioned phenyl or pyridyl radicals to be monosubstituted or polysubstituted by identical or different substituents from amongst halogen, alkyl having 1 to 4 carbon atoms, alkoxy having 1 or 2 carbon atoms, alkylthio having 1 or 2 carbon atoms, halogenoalkyl having 1 or 2 carbon atoms and 1 to 5 identical or different halogen atoms, such as fluorine atoms and chlorine atoms, halogenoalkoxy having 1 or 2 carbon atoms and 1 to 5 identical or different halogen atoms, such as fluorine atoms and chlorine atoms, halogenoalkylthio having 1 or 2 carbon atoms and 1 to 5 identical or different halogen atoms, such as fluorine atoms and chlorine atoms, cycloalkyl having 3 to 7 carbon atoms, dialkylamino having 1 to 4 carbon atoms in each alkyl part, cyano, nitro and straight-chain or branched alkoxycarbonyl having 1 to 4 carbon atoms in the alkoxy part, and

m represents the number 0 or 1,

n represents the number 0, 1 or 2,

Z represents the 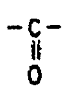 or 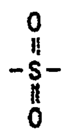 group, and

p represents the number 0 or 1,

and the acid addition salts of the hydrohalic acids, phosphoric acid, nitric acid, mono-, bi- and trifunctional carboxylic acids and hydroxycarboxylic acids and sulphonic acids, and metal salt complexes with salts of metals of main groups II to IV and of sub-groups I and II and IV to VIII of the Periodic Table of the Elements.

2. Process for the preparation of piperazinyl-methyl-1,2,4-triazolylmethylcarbinols of the formula

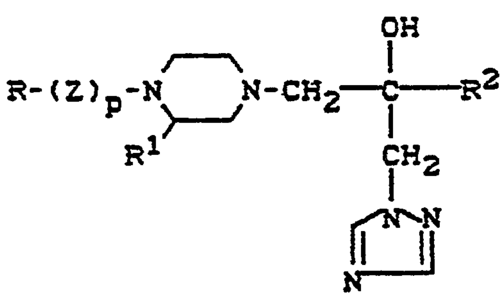

(I)

in which

R, $R^1$, $R^2$, Z and p have the meaning given in Claim 1, and of their acid addition salts and metal salt complexes according to Claim 1, characterized in that triazolylmethyloxiranes of the formula

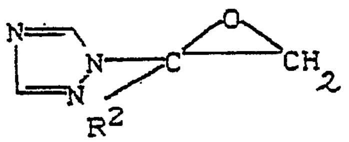

(II)

in which
$R^2$ has the meaning given above, are reacted with substituted piperazines of the formula

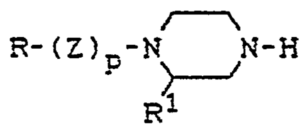

(III)

in which
R, $R^1$, Z and p have the meaning given above, if appropriate in the presence of a diluent and, if appropriate, in the presence of a catalyst, and, if required, the product is then subjected to an addition reaction with a hydrohalic acid, phosphoric acid, nitric acid, mono-, di- or tri-functional carboxylic acid, hydroxycarboxylic acid or sulphonic acid or a salt of metals of main groups II to IV or of sub-groups I or II or IV to VIII of the Periodic Table of Elements.

3. Plant protection agents, characterized in that they contain at least one piperazinylmethyl-1,2,4-triazolylmethylcarbinol of the formula (I) according to Claim 1 or an acid addition salt or metal salt complex of a piperazinylmethyl-1,2,4-triazolyl-methyl-carbinol of the formula (I) according to Claim 1.

4. Use of piperazinylmethyl-1,2,4-triazolylmethylcarbinols of the formula (I) according to Claim 1 or their acid addition salts and metal salt complexes according to Claim 1 for protecting plants.

5. Method of protecting plants, characterized in that piperazinylmethyl-1,2,4-triazolyl-methyl-carbinols of the formula (I) according to Claim 1 or their acid addition salts and metal salt complexes according to Claim 1 are applied to plants and/or their habitat.

6. Process for the preparation of plant protection agents, characterized in that piperazinylmethyl-1,2,4-triazolyl-methyl-carbinols of the formula (I) according to Claim 1 or their acid addition salts and metal salt complexes according to Claim 1 are mixed with extenders and/or surface-active substances.

7. Piperazinylmethyl-1,2,4-triazolyl-methyl-carbinol according to Claim 1 of the formula

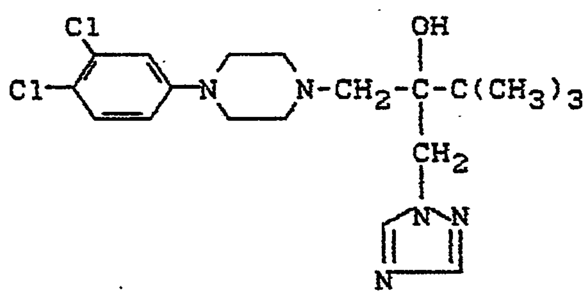

8. Piperazinylmethyl-1,2,4-triazolyl-methyl-carbinol according to Claim 1 of the formula

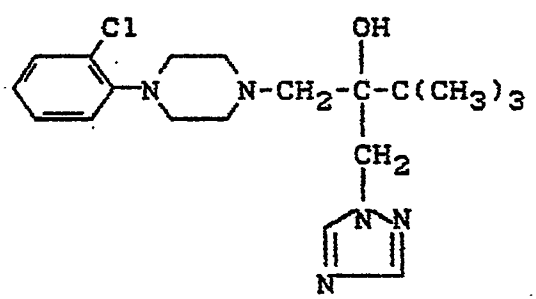

9. Piperazinylmethyl-1,2,4-triazolyl-methyl-carbinol according to Claim 1 of the formula

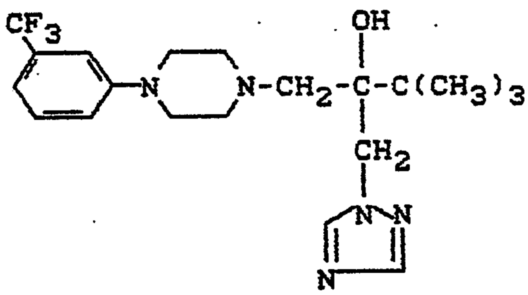

## Revendications

1. Pipérazinylméthyl-1,2,4-triazolyl-méthyl-carbinols de formule (I):

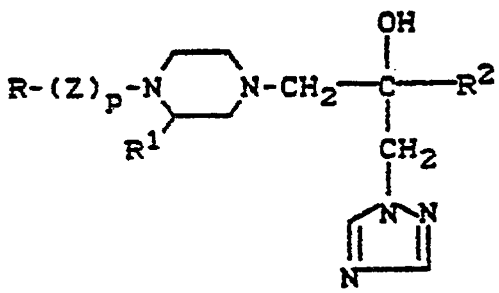

(I)

dans laquelle
R représente un groupe alkyle linéaire ou ramifié ayant 1 à 12 atomes de carbone, alcényle linéaire ou ramifié ayant 2 à 12 atomes de carbone, alcynyle linéaire ou ramifié ayant 2 à 12 atomes de carbone, alcoxyalkyle ayant 1 à 8 atomes de carbone dans la partie alkyle et 1 à 8 atomes de carbone dans la partie alcoxy, alkylthioalkyle ayant 1 à 8 atomes de carbone dans la partie alkyle et 1 à 8 atomes de carbone dans la partie alkylthio, halogénoalkyle ayant 1 à 8 atomes de carbone et 1 à 17 atomes d'halogène, identiques ou différents, un groupe halogénoalcoxyalkyle ayant 1 à 8 atomes de carbone dans la partie alkyle et 1 à 8 atomes de carbone dans la partie halogéno-alcoxy ainsi que 1 à 17 atomes d'halogène(s), identiques ou différents, halogéno-alkylthioalkyle comportant 1 à 8 atomes de carbone dans la partie alkyle et 1 à 8 atomes de carbone dans la partie halogéno-alkylthio ainsi que 1 à 17 atomes d'halogène(s), identiques ou différents, un groupe cycloalkyle comportant 3 à 7 atomes de carbone cyclique et éventuellement substitué une ou plusieurs fois, de manière identique ou différente, par un radical alkyle ayant 1 à 4 atomes de carbone, un groupe aryle ayant 6 à 10 atomes de carbone, aralkyle ayant 6 à 10 atomes de carbone dans la partie aryle et 1 à 4 atomes de carbone dans la partie alkyle, aryloxyalkyle ayant 6 à 10 atomes de carbone dans la partie aryloxy et 1 à 4 atomes de carbone dans la partie alkyle ou un groupe arylthioalkyle ayant 6 à 10 atomes de carbone dans la partie arylthio et 1 à 4 atomes de carbone dans la partie alkyle, chacun des restes aryles précités pouvant être substitué, une ou plusieurs fois, de façon identique ou différente par de l'halogène, par un groupe alkyle linéaire ou ramifié ayant 1 à 4 atomes de carbone, alcoxy ayant 1 à 4 atomes de carbone, alkylthio ayant 1 à 4 atomes de carbone, halogéno-alkyle ayant 1 à 4 atomes de carbone et 1 à 9 atomes d'halogène(s) identiques ou différents, halogéno-alcoxy ayant 1 à 4 atomes de carbone et 1 à 9 atomes d'halogène(s) identiques ou différents, halogéno-alkylthio ayant 1 à 4 atomes de carbone et 1 à 9 atomes d'halogène(s) identiques ou différents, cycloalkyle ayant 3 à 7 atomes de carbone cyclique, phényle (éventuellement substitué par de l'halogène et/ou par un groupe alkyle ayant 1 à 4 atomes de carbone), phénylalkyle comportant 1 à 4 atomes de carbone dans la partie alkyle (et éventuellement substitué par de l'halogène et/ou par un groupe alkyle ayant 1 à 4 atomes de carbone), phénylalcoxy ayant 1 à 4 atomes de carbone dans la partie alcoxy (et éventuellement substitué par de l'halogène et/ou par un groupe alkyle ayant 1 à 4 atomes de carbone) ainsi que par un groupe phénoxyalkyle ayant 1 à 4 atomes de carbone dans la partie alkyle (et éventuellement substitué par de l'halogène et/ou par un groupe alkyle ayant 1 à 4 atomes de carbone),
$R^1$ représente un atome d'hydrogène ou un groupe alkyle, linéaire ou ramifié, ayant 1 à 4 atomes de carbone,
$R^2$ représente un groupe alkyle linéaire ou ramifié ayant 1 à 8 atomes de carbone, alcényle ayant 2 à 8 atomes de carbone, un groupe cycloalkyle ayant 3 à 7 atomes de carbone cyclique (et éventuellement substitué une ou plusieurs fois par un groupe alkyle ayant 1 à 2 atomes de carbone), ainsi qu'un groupe phényle ou naphtyle, chacun des deux restes précités pouvant être substitué un ou plusieurs fois, de façon identique ou différente, par de l'halogène, par un groupe alkyle linéaire ou ramifié ayant 1 à 4 atomes de carbone ainsi que par un groupe halogéno-alkyle ayant 1 ou 2 atomes de carbone et 1 à 5 atomes d'halogène(s) identiques ou différents, comme des atomes de fluor et de chlore, et

$R^2$ représente en outre un hétérocycle pentagonal ou hexagonal, comportant éventuellement un cycle benzénique condensé, et comportant 3 hétéroatomes identiques ou différents, comme l'oxygène, l'azote et le soufre, l'hétérocycle pouvant être substitué une ou plusieurs fois, de façon identique ou différente, par de l'halogène, par un groupe alkyle ayant 1 à 4 atomes de carbone ou halogéno-alkyle ayant 1 ou 2 atome de carbone et 1 à 5 atomes d'halogène(s), identiques ou différents, comme des atomes de fluor ou de chlore, et

$R^2$ représente en outre les restes répondant aux formules

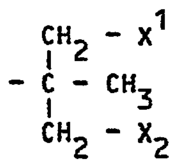

ou

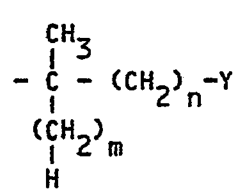

où

$X^1$ représente un atome d'hydrogène ou d'halogène,

$X^2$ représente un atome d'halogène,

Y représente un groupe alkyle, linéaire ou ramifié, ayant 1 à 6 atomes de carbone, un groupe alcoxy ayant 1 à 6 atomes de carbone, alkylthio ayant 1 à 6 atomes de carbone, halogéno-alcoxy ayant 1 à 6 atomes de carbone et 1 à 9 atomes d'halogène(s), identiques ou différents, halogéno-alkylthio ayant 1 à 6 atomes de carbone et 1 à 9 atomes d'halogène(s), identiques ou différents, alcényle ayant 2 à 6 atomes de carbone, alcoxycarbonyle linéaire ou ramifié ayant 1 à 4 atomes de carbone dans la partie alcoxy, cyano ainsi qu'un groupe phényle, phényloxy, pyridyloxy, phénylthio, phénylalcoxy ayant 1 à 4 atomes de carbone dans le groupe alcoxy et phénylalkylthio ayant 1 à 4 atomes de carbone dans le groupe alkylthio, chacun des restes phényles ou pyridyles précités pouvant être substitué une ou plusieurs fois, de façon identique ou différente, par de l'halogène, par un groupe alkyle ayant 1 à 4 atomes de carbone, alcoxy ayant 1 ou 2 atomes de carbone, alkylthio ayant 1 ou 2 atomes de carbone, halogéno-alkyle ayant 1 ou 2 atomes de carbone et 1 à 5 atomes d'halogène(s), identiques ou différents, comme des atomes de fluor et de chlore, halogéno-alcoxy ayant 1 ou 2 atomes de carbone et 1 à 5 atomes d'halogène(s), identiques ou différents, comme des atomes de fluor et de chlore, halogéno-alkylthio ayant 1 ou 2 atomes de carbone et 1 à 5 atomes d'halogène(s), identiques ou différents, comme des atomes de fluor et de chlore, cycloalkyle ayant 3 à 7 atomes de carbone, dialkylamino ayant 1 à 4 atomes de carbone dans chaque partie alkyle, cyano, nitro ainsi qu'alcoxycarbonyle linéaire ou ramifié ayant 1 à 4 atomes de carbone dans la partie alcoxy, et

m est un nombre valant 0 ou 1,

n est un nombre valant 0, 1 ou 2,

Z représente le groupe 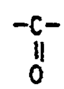 ou 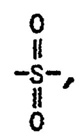, et

p est un nombre valant 0 ou 1,

ainsi que les sels d'addition d'hydracides halogénés, de l'acide phosphorique, de l'acide nitrique, d'acides carboxyliques mono-, bi- et tri-fonctionnels (acides monocarboxyliques, bicarboxyliques et tricarboxyliques), et les acides hydroxycarboxyliques ainsi que des acides sulfoniques et des complexes formés avec des sels de métaux des groupes II à IV principaux et ce groupe I et II, ainsi que IV à VIII du système au tableau périodique des éléments.

2. Procédé pour préparer des pipérazinyl-méthyl-1,2,4-triazolylméthylcarbinols de formule

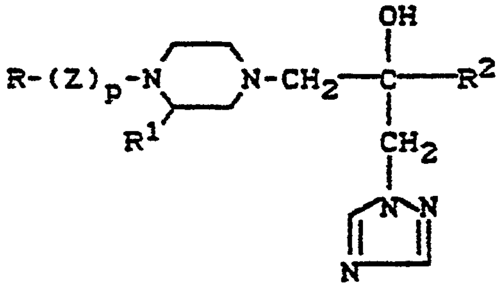

(I)

dans laquelle

R, $R^1$, $R^2$, Z et p ont le sens indiqué à la revendication1, ainsi que leurs sels d'addition d'acides et leurs complexes formés avec des sels de métaux selon la revendication 1, procédé caractérisé en ce qu'on fait réagir des triazolylméthyloxirannes de formule

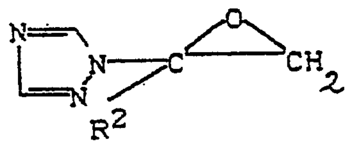

(II)

dans laquelle
$R^2$ a le sens précité, avec des pipérazines substituées de formule

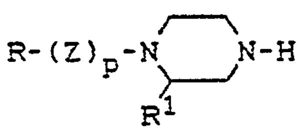

(III)

dans laquelle
R, $R^1$, Z et p ont le sens précité, en opérant éventuellement en présence d'un diluant et éventuellement en présence d'un catalyseur, et, éventuellement, on fixe ensuite par addition un hydracide halogène, de l'acide phosphorique, de l'acide nitrique, un acide carboxylique monofonctionnel, difonctionnel ou trifonctionnel, un acide hydroxycarboxylique ou un acide sulfonique, ou bien un sel de métaux des groupes principaux II à IV ou des sous-groupes I ou II ou IV à VIII du Système ou Tableau Périodique des Eléments

3. Produit phytosanitaire, caractérise par une teneur en au moins un pipérazinylméthyl-1,2,4-triazolyl-méthyl-carbinol de formule (I) selon la revendication 1 ou en un sel d'addition d'acide ou en un complexe, formé avec un sel de métal, d'un pipérazinylméthyl-1,2,4-triazolylméthyl-carbinol de formule (I) selon la revendication 1.

4. Utilisation des pipérazinylméthyl-1,2,4-triazolyl-méthyl-carbinols de formule (I) selon la revendication 1, ou de leurs sels d'addition d'acides et de leurs complexes avec des sels de métaux selon la revendication 1, pour la protection des plantes.

5. Procédé pour protéger les plantes, caractérisé en ce qu'on applique aux plantes et/ou à leur biotope ou espace vital des pipérazinylméthyl-1,2,4-triazolyl-méthyl-carbinols de formule (I) selon la revendication 1 ou leurs sels d'addition d'acides et leurs complexes avec des sels de métaux selon la revendication 1.

6. Procédé pour préparer des produits phytosanitaires pour la protection des plantes, caractérisé en ce qu'on mélange des pipérazinylméthyl-1,2,4-triazolyl-méthyl-carbinols de formule (I) selon la revendication 1 ou leurs sels d'addition d'acides et leurs complexes avec des sels de métaux selon la revendication 1, avec des agents d'allongement et/ou des substances tensioactives.

7. Pipérazinylméthyl-1,2,4-triazolyl-méthyl-carbinol selon la revendication 1, de formule

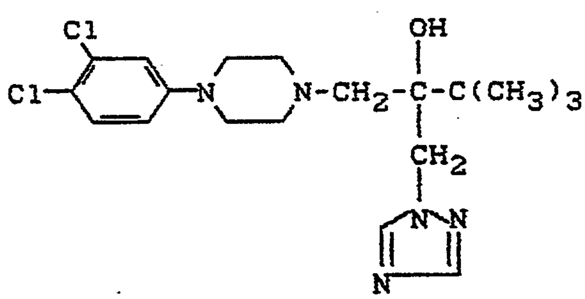

8. Pipérazinylméthyl-1,2,4,triazolyl-méthyl-carbinol selon la revendication 1, de formule

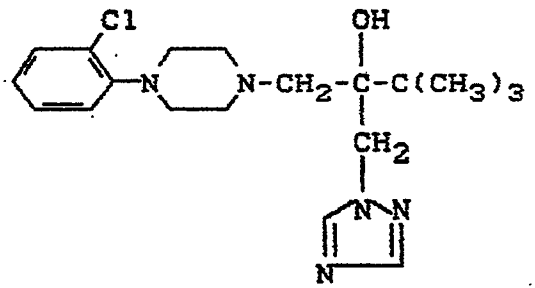

9. Pipérazinylméthyl-1,2,4,triazolyl-méthyl-carbinol selon la revendication 1, de formule